# EUROPÄISCHE PATENTSCHRIFT

(11) **EP 3 185 006 B1**
(45) Veröffentlichungstag und Bekanntmachung des Hinweises auf die Patenterteilung: **06.10.2021**
(21) Anmeldenummer: 16199865.3
(22) Anmeldetag: 21.11.2016
(51) Int. Cl.: G01N 24/08, G01R 33/38, G01R 33/44, G01N 33/22

(54) **VERWENDUNG EINES NMR-MESSGERÄTS ZUR UNTERSUCHUNG VON KRAFTSTOFF, ÖL UND/ODER HYDRAULIKFLÜSSIGKEIT**
USE OF A NMR MEASURING DEVICE FOR EXAMINATION OF FUEL, OIL AND/OR HYDRAULIC FLUID
UTILISATION D'UN APPAREIL RMN DESTINÉ À ANALYSER LE CARBURANT, L'HUILE ET/OU LE LIQUIDE HYDRAULIQUE

(30) Priorität: 21.12.2015 DE 102015226179
(43) Veröffentlichungstag der Anmeldung: 28.06.2017
(73) Patentinhaber: Robert Bosch GmbH, 70442 Stuttgart (DE)
(72) Erfinder: Krapf, Reiner, 70794 Filderstadt (DE); Marx, Klaus, 70563 Stuttgart (DE); Graessl, Andreas, 70771 Leinfelden-Echterdingen (DE); Hoffmann, Ulli, 75223 Niefern-Oeschelbronn (DE)

(56) Entgegenhaltungen:
- EP-A2- 2 182 390
- WO-A1-2014/004573
- GB-A- 2 404 026
- JP-A- 2011 080 766
- LUIS F. CABECA ET AL: "Monitoring the Transesterification Reaction Used in Biodiesel Production, with a Low Cost Unilateral Nuclear Magnetic Resonance Sensor", ENERGY & FUELS., Bd. 25, Nr. 6, 16. Juni 2011 (2011-06-16), Seiten 2696-2701, XP055371564, WASHINGTON, DC, US. ISSN: 0887-0624, DOI: 10.1021/ef200294j
- R. S. KASHAEV ET AL: "The influence of sulfur on the structural-dynamic parameters of petroleum systems studied by the NMR technique", PETROLEUM CHEMISTRY, Bd. 49, Nr. 6, 1. November 2009 (2009-11-01), Seiten 507-511, XP055371258, GB ISSN: 0965-5441, DOI: 10.1134/S0965544109060103
- MEIJUAN CHEN ET AL: "Investigation of the extraction process in gel-spinning technology for ultrahigh-molecular-weight polyethylene fibers by low-field nuclear magnetic resonance", JOURNAL OF APPLIED POLYMER SCIENCE, Bd. 132, Nr. 23, 24. Februar 2015 (2015-02-24), Seiten n/a-n/a, XP055371571, US ISSN: 0021-8995, DOI: 10.1002/app.42018
- PRYAKHIN A E ET AL: "NMR FLOWMETER FOR PROTON-BEARING LIQUIDS", MEASUREMENT TECHNIQUES, CONSULTANTS BUREAU. NEW YORK, US, Bd. 31, Nr. 11, 1. November 1988 (1988-11-01), Seiten 1092-1094, XP000053071, ISSN: 0543-1972, DOI: 10.1007/BF00864310
- WEI HE ET AL: "V-shaped portable NMR sensor with a deep penetration depth and its application in assessing the aging level of turbine oils in power stations", INTERNATIONAL JOURNAL OF APPLIED ELECTROMAGNETICS AND MECHANICS IOS PRESS NETHERLANDS, Bd. 49, Nr. 4, 2015, Seiten 567-576, XP008184503, ISSN: 1383-5416
- ZANIER A ET AL: "THERMISCH-OXIDATIVE STABILITAET VON MOTORENBENZINEN", ERDOEL ERDGAS KOHLE, URBAN VERLAG, HAMBURG, DE, Bd. 116, Nr. 3, 1. März 2000 (2000-03-01), Seiten 109-115, XP000923585, ISSN: 0179-3187

## Beschreibung

### Stand der Technik

Die vorliegende Erfindung betrifft die Verwendung eines Messgeräts, insbesondere eines handgehaltenen Messgeräts, mit einem Kernspinresonanz-Sensor.

Aus DE 10 2014 218 375 A1 und DE 10 2014 218 371 A1 sind Messgeräte mit jeweils einer Sensorvorrichtung bekannt, wobei die Sensorvorrichtung zumindest einen Kernspinresonanz-Sensor aufweist, der zur Bestimmung eines Feuchtewerts bzw. zur Detektion und/oder Analyse und/oder Unterscheidung von Materialkennwerten eines zu untersuchenden Werkstücks vorgesehen ist. Ferner ist die Verwendung eines handgehaltenen NMR-Messgeräts zur Untersuchung von Kraftstoff bekannt aus Cabeca, Energy & Fuels, Bd. 25, Nr. 6, 16. Juni 2011, Kashaev, Petroleum Chemistry, Bd. 49, Nr. 6, 1. November 2009; Chen, Journal of Applied Polymer Science, Bd. 132, Nr. 23, 24. Februar 2015; WO 2014/004573 A1; JP 2011 080766 A; GB 2 404 026 A; Pryakhin, Measurement Techniques, Consultants Bureau, New York, US, Bd. 31, Nr. 11, 1. November 1988. Aus EP 2 182 390 A2 ist bekannt, eine möglichst kompakte Vorrichtung zur Verfügung zu stellen, welche ohne externe Komponenten auskommt.

### Offenbarung der Erfindung

Erfindungsgemäß wird die Verwendung eines Messgeräts, insbesondere eines handgehaltenen Messgeräts, bevorzugt eines Messgeräts mit einem Gehäuse, mit zumindest
- einem Kernspinresonanz-Sensor,
- einer Steuervorrichtung zur Steuerung des Messgeräts,
- einer Auswertevorrichtung zur Auswertung eines von dem Kernspinresonanz-Sensor gelieferten Messsignals,
- einer Ausgabevorrichtung zur Ausgabe von ermittelten Informationen sowie
- einer Energieversorgungsvorrichtung
zur Untersuchung eines Kraftstoffs und/oder eines Öls und/oder einer Hydraulikflüssigkeit vorgeschlagen. Die erfindungsgemässe Verwendung ist in Anspruch 1 definiert.

In einer Ausführungsform stellt das Messgerät ein handgehaltenes Messgerät dar. Unter einem "handgehaltenen Messgerät" soll hier insbesondere verstanden werden, dass das Messgerät ohne Zuhilfenahme einer Transportmaschine lediglich mit den Händen, insbesondere mit einer Hand, transportiert werden kann. Insbesondere kann das Messgerät auch während eines Messvorgangs an ein und/oder entlang eines zu untersuchenden Kraftstoffs und/oder Öls und/oder einer Hydraulikflüssigkeit bzw. an und/oder entlang eines Behältnisses (im Folgenden nicht wiederholt ausgeführt), in dem die entsprechende Flüssigkeit enthalten ist, geführt werden kann. Dazu beträgt die Masse des handgehaltenen Messgeräts insbesondere weniger als 20 kg, vorteilhaft weniger als 10 kg und besonders vorteilhaft weniger als 2 kg. In einer Ausführungsform weist das Messgerät einen Griff oder einen Griffbereich auf, mit dem das Messgerät über einen zu untersuchenden Kraftstoff und/oder ein zu untersuchendes Öl und/oder eine zu untersuchende Hydraulikflüssigkeit bzw. ein entsprechendes Behältnis geführt werden kann. Alternativ oder zusätzlich kann auch das zu untersuchende Öl und/oder der Kraftstoff und/oder die Hydraulikflüssigkeit bzw. ein entsprechendes Behältnis zur Untersuchung an das Messgerät und/oder entlang des Messgeräts geführt werden.

Gemäss einem Aspekt der Erfindung wird das Messgerät in einem Fahrzeug, insbesondere in einem Kraftfahrzeug, verwendet. Ein derartiges Fahrzeug ist in Anspruch 8 definiert. Das Messgerät kann sowohl handgehalten als auch stationär, beispielsweise fest in das Fahrzeug integriert bzw. eingebaut, ausgeführt sein.

In einer Ausführungsform des Messgeräts sind die Komponenten des Messgeräts, insbesondere der Kernspinresonanz-Sensor, die Steuervorrichtung, die Auswertevorrichtung sowie die Energieversorgungsvorrichtung des Messgeräts, zumindest teilweise in dem Gehäuse des Messgeräts untergebracht. Insbesondere sind die Komponenten in ihrem Gesamtvolumen zu mehr als 50 %, bevorzugt zu mehr als 75 % und besonders bevorzugt zu 100 % in dem Gehäuse des Messgeräts untergebracht. Bei einem derart gestalteten Messgerät kann eine vorteilhafte Verwendung, insbesondere durch leichtes Führen des Messgeräts im Falle eines handgehaltenen Messgeräts, realisiert sein. Des Weiteren sind die Komponenten auf diese Weise vor Beschädigungen und Umwelteinflüssen, beispielsweise Feuchtigkeit und Staub, geschützt.

Das Messgerät weist eine Energieversorgungsvorrichtung auf, die dazu vorgesehen ist, das Messgerät zur Inbetriebnahme und während des Betriebs mit elektrischer Energie zu versorgen. In einer Ausführungsform ist die Energieversorgungsvorrichtung als eine kabelgebundene Stromversorgung unter Verwendung einer externen Energiequelle realisiert, beispielsweise in Form eines elektrischen Anschlusses an einen Stromkreis, an eine Batterie, insbesondere eine Fahrzeugbatterie, an eine Lichtmaschine oder dergleichen. In dieser Ausführungsform stellt die Steckverbindung des Messgeräts zur Verbindung mit der externen Energiequelle, insbesondere auch in Verbindung mit einem Transformator, eine Energieversorgungsvorrichtung dar. In einer Ausführungsform dieser Steckverbindung ist das Messgerät mittels einer Formschluss- und/oder Kraftschlussverbindungsschnittstelle der Steckverbindung von der externen Energiequelle lösbar. Unter "lösbar" soll in diesem Zusammenhang insbesondere zerstörungsfrei trennbar verstanden werden. Somit ist das Messgerät bevorzugt von der externen Energiequelle abnehmbar und/oder abkoppelbar.

In einer alternativen oder zusätzlichen Ausführungsform ist das Messgerät, insbesondere das handgehaltene Messgerät, als ein energieautonomes Messgerät realisiert. Unter "energieautonom" ist zu verstehen, dass das Messgerät zumindest vorübergehend, bevorzugt zumindest während der Dauer einer Untersuchung eines Kraftstoffs und/oder Öls und/oder einer Hydraulikflüssigkeit, unabhängig von einem Stromnetz, d.h. insbesondere kabellos, betrieben werden kann. Dazu kann das Messgerät eine Energieversorgungsvorrichtung in Form eines stromnetzunabhängigen Energiespeichers, insbesondere in Form einer Batterie, bevorzugt in Form einer wiederaufladbaren Batterie, aufweisen. Der stromnetzunabhängige Energiespeicher kann in einer alternativen Ausführungsform als eine Brennstoffzelle, ein Kondensator, ein hybrider Superkondensator oder als ein anderweitiger, dem Fachmann sinnvoll erscheinender Energiespeicher oder eine Kombination/Mehrung derer realisiert sein. Insbesondere eignen sich zur Energieversorgung des Messgeräts Akkumulatoren mit einer Zellchemie, die eine hohe Leistungs- und/oder Energiedichte bereitstellt. Eine hohe Leistungs- und/oder Energiedichte erlaubt eine verbesserte, d.h. langlebigere und an einen hohen Leistungsbedarf des Kernspinresonanz-Sensors angepasste, Energieversorgung des Messgeräts. Dazu gehören derzeit beispielsweise Akkumulatoren der Lithium- und Lithium-Ionen-Zellchemie, insbesondere Lithium-Eisenphosphat-, Lithium-Manganoxid-, Lithium-Nickel-Cobalt-Mangan-Oxid-, überlithiierte Lithium-Nickel-Cobalt-Mangan-Oxid-, Lithium-Schwefel-, Lithium-Polymer- und Lithium-Sauerstoff-Akkumulatoren. In dieser Ausführungsform kann die Energieversorgungsvorrichtung mittels einer Formschluss- und/oder Kraftschlussverbindungsschnittstelle vom Messgerät lösbar realisiert sein. Unter "lösbar" soll in diesem Zusammenhang insbesondere zerstörungsfrei trennbar verstanden werden. Somit ist die Energieversorgungsvorrichtung bevorzugt abnehmbar und austauschbar an und/oder in dem Messgerät anordenbar. In Form eines derartigen Energiespeichers lässt sich die abnehmbare Energieversorgungsvorrichtung in und/oder außerhalb des Messgeräts wieder mit Energie aus einem Stromnetz versorgen und laden. In einer Ausführungsform der Energieversorgungsvorrichtung ist diese dazu vorgesehen, neben der Verwendung zur Energieversorgung des Messgeräts auch zur Energieversorgung anderer Geräte, insbesondere anderer Messgeräte und/oder anderer Handwerkzeugmaschinenvorrichtungen, nutzbar zu sein.

Unter "vorgesehen" soll insbesondere speziell "programmiert", "ausgelegt" und/oder "ausgestattet" verstanden werden. Darunter, dass ein Objekt zu einer bestimmten Funktion "vorgesehen" ist, soll insbesondere verstanden werden, dass das Objekt diese bestimmte Funktion in zumindest einem Anwendungs- und/oder Betriebszustand erfüllt und/oder ausführt oder dazu ausgelegt ist, die Funktion zu erfüllen.

Das Messgerät weist eine Steuervorrichtung zu dessen Steuerung auf. Die Steuervorrichtung ist mit den anderen Komponenten des Messgeräts, insbesondere dem Kernspinresonanz-Sensor, der Auswertevorrichtung, der Ausgabevorrichtung, ferner beispielsweise auch mit einer Eingabevorrichtung, der Energieversorgungsvorrichtung und/oder einer Datenkommunikationsschnittstelle, signaltechnisch verbunden. Die Steuervorrichtung ist dazu vorgesehen, während des Betriebs des Messgeräts mit diesen Komponenten zu kommunizieren. Unter der "Steuervorrichtung" soll insbesondere eine Vorrichtung mit zumindest einer Steuerelektronik verstanden werden, die Mittel zur Kommunikation mit den anderen Komponenten des Messgeräts, beispielsweise Mittel zur Steuerung und/oder Regelung des Kernspinresonanz-Sensors, Mittel zur Datenverarbeitung, Mittel zur Datenspeicherung und/oder weitere, dem Fachmann als sinnvoll erscheinende Mittel aufweist. In einer Ausführungsform ist unter der Steuerelektronik der Steuervorrichtung eine Prozessoreinheit in Verbindung mit einer Speichereinheit sowie mit einem in der Speichereinheit gespeicherten Betriebsprogramm zu verstehen, das während des Steuervorgangs ausgeführt wird. Insbesondere können die elektronischen Bauteile der Steuervorrichtung auf einer Platine (Leiterplatte) angeordnet sein, beispielsweise in Form eines Mikrokontrollers.

Zur Untersuchung eines Kraftstoffs und/oder Öls und/oder einer Hydraulikflüssigkeit weist das Messgerät einen Kernspinresonanz-Sensor auf. Die Funktionsweise des Kernspinresonanz-Sensors basiert auf dem kernphysikalischen Effekt, bei dem Atomkerne in dem zu untersuchenden Öl und/oder Kraftstoff in einem ersten Magnetfeld, bezeichnet mit Bo, elektromagnetische Wechselfelder absorbieren und emittieren. Dabei beruht die Kernspinresonanz auf der Präzession (Larmorpräzession) von Kernspins der Atomkerne in dem zu untersuchenden Medium um die Magnetfeldlinien des ersten, insbesondere konstanten und/oder statischen, Magnetfelds. Insbesondere werden die Kernspins der Atomkerne in dem zu untersuchenden Öl und/oder Kraftstoff und/oder Hydraulikflüssigkeit durch das erste Magnetfeld ausgerichtet. Wird Energie in Form eines zweiten elektromagnetischen Feldes, insbesondere eines Wechselfeldes, beispielsweise eines gepulsten Magnetfeldes, auf die Atomkerne eingestrahlt, die mit der Larmorpräzession deren Kernspins in Resonanz ist (Energiequanten), so können die Atomkerne die Orientierung ihrer Spins relativ zum ersten Magnetfeld durch Absorption dieser Energie ändern. Das zweite eingestrahlte Magnetfeld dient daher der Anregung der Kernspins, die unter Energieaufnahme ihre Kernspinzustände ändern. Äquivalent führt die Emission von Energiequanten in Folge einer Rückkehr der angeregten Kernspins in ein anderes, niedrigeres Energieniveau, zur Emission eines elektromagnetischen Wechselfeldes, welches sich mittels einer Vorrichtung zur Detektion einer Magnetfeldänderung, insbesondere mittels einer Antenne und/oder einer Spule, beobachten lässt. Unter den Atomkernen sind insbesondere Protonen (H) und andere kernspinresonanz-aktive Kerne wie beispielsweise 13C, 15N, 19F, 31P zu verstehen.

Der Kernspinresonanz-Sensor des Messgeräts erlaubt somit, Atomkerne in dem zu untersuchenden Öl und/oder Kraftstoff und/oder der zu untersuchenden Hydraulikflüssigkeit mittels elektromagnetischer Wechselfelder anzuregen sowie ein Ausgangssignal aufgrund eines Kernspinresonanzeffektes zu generieren. Unter Anregung von Atomkernen soll insbesondere verstanden werden, dass die Energie der eingestrahlten elektromagnetischen Felder, insbesondere Wechselfelder, eine Änderung der Kernspins der Atomkerne bewirkt. Es sei angemerkt, dass im Folgenden davon ausgegangen wird, dass insbesondere veränderliche Magnetfelder mit elektrischen Feldern gekoppelt sind (vgl. Maxwell-Gleichungen), sodass keine Unterscheidung zwischen elektrischem Feld und Magnetfeld vorgenommen wird. Zur Anregung von Kernspinresonanz-Effekten kommt es auf die durch eine eingestrahlte elektromagnetische Strahlung übertragene Energie an. In einer Ausführungsform lässt sich diese Energie mittels gepulster elektromagnetischer Felder übertragen.

Bei geeigneter Wahl der Betriebsparameter des Kernspinresonanz-Sensors kann unter Verwendung des Messgeräts mittels des gemessenen Antwortsignals bei geeigneter Auswertung unmittelbar auf das Öl und/oder den Kraftstoff und/oder die Hydraulikflüssigkeit sowie auf dessen/deren Eigenschaften, zumindest in einem untersuchten Volumen, geschlossen werden. Die Eigenschaften können dabei beispielsweise die Qualität, Herkunft, Echtheit oder dergleichen des Kraftstoffs und/oder Öls und/oder der Hydraulikflüssigkeit betreffen.

Unter der "Auswertevorrichtung" zur Auswertung zumindest eines von dem Kernspinresonanz-Sensor gelieferten Messsignals soll zumindest eine Vorrichtung verstanden werden, die einen Informationseingang zur Annahme der Messsignale des Kernspinresonanz-Sensors, eine Informationsverarbeitungseinheit zur Bearbeitung, insbesondere Auswertung der angenommenen Messsignale, sowie eine Informationsausgabe zur Weitergabe der bearbeiteten und/oder ausgewerteten Messsignale aufweist. In einer Ausführungsform weist die Auswerteeinheit Komponenten auf, die zumindest einen Prozessor, einen Speicher und ein Betriebsprogramm mit Auswerte- und Berechnungsroutinen umfassen. Insbesondere können die elektronischen Bauteile der Auswertevorrichtung auf einer Platine (Leiterplatte) angeordnet sein, insbesondere auf einer gemeinsamen Platine mit der Steuervorrichtung. In einer Ausführungsform kann die Auswertevorrichtung in Form eines Mikrokontrollers realisiert sein. Des Weiteren können die Steuervorrichtung und die Auswertevorrichtung als ein einzelnes Bauteil ausgeführt sein. Die Auswertevorrichtung ist vorgesehen, die von dem Kernspinresonanz-Sensor erhaltenen Messsignale auszuwerten und daraus zumindest eine Information betreffend das untersuchte Öl und/oder den untersuchten Kraftstoff und/oder die untersuchte Hydraulikflüssigkeit abzuleiten.

Unter der "Ausgabevorrichtung" des Messgeräts soll zumindest eine Komponente des Messgeräts verstanden werden, welche der Kommunikation des Messgeräts mit einer externen Umgebung, beispielsweise mit externen Geräten, Menschen oder dergleichen dient. Bei der Kommunikation erfolgt eine Ausgabe von Informationen in Form von elektrischer Spannung, Stromstärke, akustischem Schall, elektromagnetischen Wellen, Daten oder dergleichen. Derart kann die Ausgabevorrichtung insbesondere als Datenschnittstelle (im Folgenden als "Datenkommunikationsschnittstelle" bezeichnet) und/oder als Benutzerschnittstelle zwischen Mensch und Gerät (im Folgenden als "Ausgabevorrichtung zur Ausgabe an einen Bediener" des Messgeräts bezeichnet) realisiert sein.

In einer Ausführungsform des Messgeräts, insbesondere im Falle eines handgehaltenen Messgeräts, ist unter der Ausgabevorrichtung beispielsweise ein Mittel zu verstehen, das dazu vorgesehen ist, zumindest eine wechselnde Information akustisch, optisch und/oder taktil an einen Bediener auszugeben. Die Ausgabevorrichtung dient dabei der Ausgabe zumindest derjenigen Informationen an den Bediener des Messgeräts, die unter Verwendung des Messgeräts über das untersuchte Öl und/oder über den untersuchten Kraftstoff und/oder über die untersuchte Hydraulikflüssigkeit erhalten werden. Die Ausgabe kann dabei beispielsweise mittels eines Displays, eines Touch-Displays, eines Tonsignals, eines Vibrationsgebers und/oder einer LED-Anzeige realisiert werden. In einer Ausführungsform der Ausgabevorrichtung zur Ausgabe an einen Bediener kann die Information grafisch oder alphanumerisch als Messergebnis der Untersuchung ausgegeben werden. Die Ausgabevorrichtung ist in einer Ausführungsform in einem Gehäuse des Messgeräts untergebracht.

Ferner können auszugebende Informationen, beispielsweise Informationen über ein untersuchtes Öl und/oder Kraftstoff und/oder über eine untersuchte Hydraulikflüssigkeit, auch an die Steuervorrichtung und/oder, insbesondere zur Erhöhung des Nutzerkomforts, an ein Daten verarbeitendes System ausgegeben werden. Letzteres umfasst zumindest eine Ausgabe einer Information an ein externes Gerät wie ein Smartphone, ein Tablet-PC, ein PC sowie an ein anderes, einem Fachmann als sinnvoll erscheinendes externes Datengerät wie beispielsweise eine Motorsteuerung oder dergleichen, das über eine Datenkommunikationsschnittstelle mit der Auswertevorrichtung und/oder der Steuervorrichtung des Messgeräts verbunden ist. Die Datenkommunikationsschnittstelle dient dabei der, insbesondere drahtlosen, Kommunikation, mittels der das Messgerät Messsignale, ausgewertete Informationen und/oder Arbeitsparameter senden und/oder empfangen kann. Bevorzugt verwendet die Datenkommunikationsschnittstelle ein standardisiertes Kommunikationsprotokoll zu einer Übertragung von elektronischen, insbesondere digitalen Daten. In einer Ausführungsform umfasst die Datenkommunikationsschnittstelle eine drahtlose Schnittstelle, insbesondere beispielsweise eine WLAN-, Bluetooth-, Infrarot-, NFC-, RFID-, GSM-Schnittstelle oder eine andere, einem Fachmann als sinnvoll erscheinende drahtlose Schnittstelle. Alternativ kann die Datenkommunikationsschnittstelle auch einen kabelgebunden Adapter aufweisen, beispielsweise einen Netzwerk-, USB- oder Mikro-USB-Adapter. Ferner kann unter Verwendung der Datenkommunikationsschnittstelle eine Übertragung von Referenzdaten ermöglicht werden, die zu einer Auswertung und/oder Interpretation von mit dem Messgerät erfassten Messsignalen nutzbar sein können. Insbesondere werden die Referenzdaten dabei von einer geräteinternen und/oder einer geräteexternen Referenzdatenbank abgerufen. Ferner können vorteilhaft vielfältige Zusatzfunktionen ermöglicht und eingebunden werden, die insbesondere auch eine direkte Kommunikation mit Smartphones (insbesondere über programmierte Apps) oder ähnlichen portablen Datengeräten erfordern. Diese können beispielsweise automatische Kartierungs-Funktionen, Firmware-Updates, Datennachbearbeitung, Datenaufbereitung, Datenabgleich mit anderen Geräten, etc. umfassen.

Somit kann die Ausgabevorrichtung direkt im Gehäuse des Messgeräts, insbesondere im Falle eines handgehaltenen Messgeräts, untergebracht sein und/oder über externe Ausgabevorrichtungen, insbesondere im Falle eines stationären Messgeräts, realisiert bzw. ergänzt werden. Letztere Realisierungsmöglichkeit umfasst explizit die Steuerung, Auswertung und Ausgabe der ermittelten Informationen an oder über drahtgebundene und/oder drahtlose externe Systeme wie beispielsweise Fernbedienungen, Computersteuerungen, Tablet-PCs, Mobiltelefone, Smartphones und/oder andere Geräte wie Steuergeräte oder dergleichen.

Unter "Öl" sollen entsprechend chemischer Definition insbesondere jegliche Form von Flüssigkeiten verstanden werden, die sich nicht mit Wasser mischen lassen. In einer Ausführungsform können dies insbesondere Mineralöl, Erdöl, Heizöl, Dieselöl, Schweröl, synthetisches Öl oder dergleichen sein. Insbesondere sind unter Öl auch Hydraulikflüssigkeiten wie beispielsweise Bremsflüssigkeit sowie Schmiermittel zu verstehen, wobei letzteres insbesondere dazu geeignet ist, mechanische Komponenten wie beispielsweise einen Verbrennungsmotor zu schmieren. Unter "Kraftstoff" soll hier ein flüssiger oder gasförmiger Brennstoff zum Antrieb von Fahrzeugen - beispielsweise von Kraftfahrzeugen, Flugzeugen, Schiffen oder dergleichen - verstanden werden. Der Kraftstoff dient dabei der Bereitstellung von chemisch gebundener Energie. Die chemisch gebundene Energie kann dabei in Folge von Verbrennung - beispielsweise in Verbrennungskraftmaschinen wie Verbrennungsmotoren oder dergleichen - in mechanische Energie umgewandelt werden. Typische Beispiele für einen derartigen Kraftstoff sind Diesel, Benzin, Kerosin, Ethanol-Kraftstoff, Flüssigerdgas, Flüssiggas, Benzol oder dergleichen.

Unter "Verwendung zur Untersuchung eines Kraftstoffs und/oder Öls und/oder einer Hydraulikflüssigkeit" soll insbesondere verstanden werden, unter Verwendung des Messgeräts aus den von dem Kernspinresonanz-Sensor erhaltenen Messsignalen Informationen zu bestimmen und somit Aussagen abzuleiten, die die Eigenschaften und insbesondere den Zustand eines Öls und/oder eines Kraftstoffs und/oder einer Hydraulikflüssigkeit betreffen. Insbesondere lassen sich bei geeigneter Auswertung der Messsignale des Kernspinresonanz-Sensors Informationen ermitteln hinsichtlich Herkunft (Zusammensetzung, chemische Behandlung), hinsichtlich Qualität (Wasserverteilung, Wassergehalt, Alterungszustand, Verunreinigungen, Zusammensetzung, Viskosität, Porosität), hinsichtlich Inhaltsstoffe (gesundheitsschädliche Inhaltsstoffe, verbotene Inhaltsstoffe, Metallgehalt, deklarierte Inhaltsstoffe) oder dergleichen. Aus diesen ausgewerteten Informationen (im Folgenden auch "Auswerteergebnisse") kann ein Bediener des Messgeräts und/oder ein von dem Messgerät mit Informationen versorgtes externes Gerät somit die Qualität, Herkunft, Echtheit, Viskosität, Porosität oder dergleichen des Kraftstoffs und/oder des Öls und/oder der Hydraulikflüssigkeit auf einfache Weise untersuchen und prüfen. Ferner lassen sich die erhaltenen Informationen mit Herstellerangaben zu dem untersuchten Öl und/oder Kraftstoff und/oder der untersuchten Hydraulikflüssigkeit vergleichen und somit deren Authentizität prüfen. Manipulationen, Fälschungen, Falschetikettierungen oder dergleichen lassen sich auf einfache Weise durch eine kurze Untersuchung des Kraftstoffs und/oder Öls und/oder der Hydraulikflüssigkeit aufdecken.

Zur Durchführung der Messung wird das Messgerät, insbesondere das handgehaltene Messgerät, insbesondere dessen Kernspinresonanz-Sensor, nahe an das zu untersuchende Öl und/oder an den zu untersuchenden Kraftstoff und/oder an die zu untersuchende Hydraulikflüssigkeit (oder deren/dessen Behältnis) herangebracht oder umgekehrt. Die Verwendung des Messgeräts erlaubt dabei die Untersuchung ohne Beeinträchtigung des Kraftstoffs und/oder Öls und/oder der Hydraulikflüssigkeit, d.h. insbesondere ohne Zerstörung, Kontamination oder dergleichen. Insbesondere handelt es sich bei dem Kernspinresonanz-Messverfahren um ein nicht zerstörendes, insbesondere kontaktloses Messverfahren, sodass ein Öl und/oder ein Kraftstoff und/oder eine Hydraulikflüssigkeit ohne jeglichen Kontakt zu dem Messgerät untersucht werden kann. Das Positionieren des Messgerätes, insbesondere des darin enthaltenen Kernspinresonanz-Sensors, in unmittelbare Nähe eines zu untersuchenden Kraftstoffs und/oder Öls und/oder einer zu untersuchenden Hydraulikflüssigkeit (oder umgekehrt) ermöglicht die Untersuchung bis zu einer Materialtiefe von einigen Zentimeter in das Öl und/oder in den Kraftstoff und/oder in die Hydraulikflüssigkeit hinein. Somit kann ebenfalls ein verpacktes/eingeschlossenes Öl und/oder Kraftstoff und/oder Hydraulikflüssigkeit ohne Zerstörung bzw. Öffnen des Behältnisses untersucht werden. In einer Ausführungsform kann ebenfalls ein Öl und/oder ein Kraftstoff und/oder eine Hydraulikflüssigkeit in einem abgeschlossenen System, beispielsweise in einem Schlauchsystem eines Kreislaufs in einem Motor, ohne Zerstörung oder Öffnen des Systems untersucht werden.

Das Messgerät, insbesondere das handgehaltene Messgerät, stellt ein spezialisiertes Messgerät dar, das im Vergleich zu wissenschaftlichen Kernspinresonanz-Messgeräten eine stark eingeschränkte, auf die Untersuchung eines Kraftstoffs und/oder eines Öls und/oder einer Hydraulikflüssigkeit optimierte Funktionalität aufweist. Insbesondere die Auswertevorrichtung mit ihren Auswerteroutinen ist auf die Untersuchung von Öl und/oder Kraftstoff und/oder Hydraulikflüssigkeit, die Bewertung der erhaltenen Informationen sowie deren aufbereiteter Ausgabe mittels der Ausgabevorrichtung zugeschnitten. Bei Verwendung des Messgeräts zur Untersuchung von Kraftstoff und/oder Öl und/oder Hydraulikflüssigkeit werden die Messergebnisse, d.h. die gewonnenen Informationen, für den Bediener des Messgeräts und/oder für ein externes weiteres Gerät (beispielsweise eine Motorsteuerung oder eine Bremskraftverstärkersteuerung) geräteintern und der Messung unmittelbar folgend aufbereitet, sodass eine schnelle, eindeutige und vor allem von weiteren Geräten wie Computern oder gar von Laboratorien unabhängige Beurteilung des untersuchten Kraftstoffs und/oder Öls und/oder der untersuchten Hydraulikflüssigkeit vor Ort möglich ist. Vorteilhaft ist eine einfache und intuitive Bedienung des Messgeräts erreichbar, die keine besondere Vorerfahrung des Bedieners voraussetzt.

Darüber hinaus sind die Baugröße, die Energieversorgung sowie die Konstruktion des Messgeräts hinsichtlich Anordnung des Kernspinresonanz-Sensors an die Verwendung des Messgeräts zur Untersuchung von Öl und/oder Kraftstoff und/oder Hydraulikflüssigkeit angepasst. So weist das Messgerät in einer Ausführungsform, insbesondere im Falle eines handgehaltenen Messgeräts, beispielsweise eine plane Auflagefläche auf, an die ein Öl und/oder Kraftstoff und/oder eine Hydraulikflüssigkeit zur Untersuchung angelegt werden kann. In einer alternativen oder zusätzlichen Ausführungsform des handgehaltenen Messgeräts weist dieses in dem Gehäuse eine Aufnahme für eine Öl- und/oder Kraftstoff- und/oder Hydraulikflüssigkeitsprobe auf, beispielsweise für ein Behältnis in Form einer Dose, einer Flasche und/oder eines Probenröhrchens. Diese Aufnahme kann in einer Ausführungsform rund und/oder zylindrisch und/oder C-förmig ausgestaltet sein.

In einer Ausgestaltung wird das Messgerät zur Untersuchung eines Kraftstoffs und/oder Öls und/oder einer Hydraulikflüssigkeit in einem Kraftfahrzeug, bevorzugt während eines Betriebs des Fahrzeugs, verwendet. Auf diese Weise kann mittels des in einem Fahrzeug integrierten Messgeräts eine Information betreffend ein Öl und/oder einen Kraftstoff und/oder eine Hydraulikflüssigkeit während des Betriebs des Fahrzeug ermittelt werden. In einer Ausführungsform kann beispielsweise eine Untersuchung des Öls und/oder des Kraftstoffs in Echtzeit erfolgen. In einer Ausführungsform des Messgeräts erfolgt die Untersuchung, d.h. das Messen, Auswerten und Ausgeben der erhaltenen Information, vollautomatisch.

In einer Ausgestaltung wird das Messgerät zur Untersuchung eines Kraftstoffs und/oder eines Öls und/oder einer Hydraulikflüssigkeit unmittelbar an einem kraftstoffführenden und/oder ölführenden und/oder hydraulikflüssigkeitführenden Bauteil des Fahrzeugs verwendet. Dazu kann das Messgeräts vorteilhaft zur Untersuchung von Öl und/oder Kraftstoff und/oder Hydraulikflüssigkeit in einem Rohr oder Schlauch oder in einem anderen kraftstoffführenden und/oder ölführenden und/oder hydraulikflüssigkeitführenden Bauteil eines Fahrzeugs vorgesehen sein. Beispielsweise kann durch eine zylindrische Aufnahme des Messgeräts, die das Messgerät vollständig durchdringt und die mit dem sensitiven Bereich des Kernspinresonanz-Sensors zusammenfällt, ein Rohr oder ein Schlauch gelegt sein. Derart wird gleichzeitig das Rohr oder der Schlauch gegen Verrutschen in dem Messgerät gesichert. Ferner kann der Kernspinresonanz-Sensor auch direkt am oder im Motor, an Anbauaggregaten des Motors oder im Ölmessstab integriert sein und auf diese Weise eine Untersuchung des Öls und/oder Kraftstoffs und/oder einer Hydraulikflüssigkeit unmittelbar am Einsatzort ermöglichen.

Durch Verwendung des speziell auf den Anwendungsfall der Untersuchung von Öl und/oder Kraftstoff und/oder Hydraulikflüssigkeit zugeschnittenen Messgeräts ist es möglich, auf schnelle und zerstörungsfreie Weise und damit wirtschaftlich besonders kostengünstig eine präzise und umfassende Untersuchung eines Kraftstoffs und/oder Öls und/oder einer Hydraulikflüssigkeit vor Ort, beispielsweise mobil in einer Werkstatt, in einem Lagerhaus, in einem Einkaufsladen, aber auch stationär in einem Motorraum oder dergleichen, zu realisieren. Vorteilhaft ermöglicht die erfindungsgemäße Verwendung des Messgeräts die schnelle und präzise Untersuchung von Öl und/oder Kraftstoff und/oder Hydraulikflüssigkeit an beliebigen Orten, d.h. insbesondere unabhängig von einem auf Kernspinresonanzspektroskopie spezialisierten Labor.

Das Messgerät wird zur Untersuchung eines Kraftstoffs und/oder Öls und/oder einer Hydraulikflüssigkeit verwendet, wobei mittels der Auswertevorrichtung des Messgeräts das von dem Kernspinresonanz-Sensor gelieferte Messsignal, insbesondere ein Spektrum und/oder eine Relaxationszeit des Messsignals resultierend aus der Anregung von Kernspins in dem zu untersuchenden Öl und/oder Kraftstoff und/oder der zu untersuchenden Hydraulikflüssigkeit durch den Kernspinresonanz-Sensor, ausgewertet wird.

Erfindungsgemäß wird das Messgerät dazu verwendet, ein Öl und/oder einen Kraftstoff und/oder eine Hydraulikflüssigkeit insbesondere hinsichtlich verschiedener Merkmale wie beispielsweise Wasserverteilung, Wassergehalt, Alterungszustand, Verunreinigungen, Zusammensetzung, Viskosität oder dergleichen umfassend zu charakterisieren. Dabei lassen sich bei Verwendung des Messgeräts mittels des Kernspinresonanz-Sensors Spektren und/oder Relaxationszeiten messen, die eine von dem untersuchten Öl und/oder Kraftstoff und/oder der Hydraulikflüssigkeit abhängige Signatur bzw. Charakteristik aufweisen, genauer gesagt, eine von deren/dessen atomaren Aufbau abhängige Signatur bzw. Charakteristik aufweisen. Die Auswertevorrichtung ist speziell zur zügigen Auswertung der von dem Kernspinresonanz-Sensor gelieferten Messsignale ausgebildet. Zügig bedeutet insbesondere innerhalb von 10 Minuten, bevorzugt innerhalb von 60 Sekunden, besonders bevorzugt innerhalb von 5 Sekunden.

Bei geeigneter Wahl der Betriebsparameter des Kernspinresonanz-Sensors kann mittels eines Spektrums und/oder Relaxationszeiten des Antwortsignals unmittelbar auf Eigenschaften des zu untersuchenden Kraftstoffs und/oder Öls und/oder der zu untersuchenden Hydraulikflüssigkeit geschlossen werden. In einem Chemometrie-Ansatz kann dabei beispielsweise durch Auswertung mittels einer Hauptkomponentenanalyse (PCA) eine Untersuchung des Kraftstoffs und/oder Öls und/oder der Hydraulikflüssigkeit erfolgen, ohne konkrete Ursache-Wirkungszusammenhänge zu kennen. Dabei werden das Spektrum oder mehrere Spektren, chemische Verschiebungen, Kopplungskonstanten, Korrelationen und/oder Relaxationszeiten oder dergleichen als Eingangsdaten der Auswertung verwendet. In Folge der Auswertung werden Punktwolken bzw. grafisch darstellbare Bereiche erhalten, die sich durch Vergleich mit Referenzdaten auf einfache und zügige Weise weiter auswerten und insbesondere interpretieren lassen. Beispielsweise kann in einer Verwendungsform des Messgerät mittels der Auswertevorrichtung aus den mit dem Kernspinresonanz-Sensor erhaltenen Messsignalen zumindest eine Information aus einer Liste von Informationen ausgewertet, wobei die Liste zumindest
- einen relativen und/oder absoluten Kohlenwasserstoffgehalt,
- Bindungszustände chemischer Verbindungen,
- einen Konzentrationsgradienten eines Materials und/oder einer Verbindung und/oder eines Elements in das Öl und/oder in den Kraftstoff und/oder in die Hydraulikflüssigkeit hinein,
- zeitlich-dynamische Prozesse chemischer Verbindungen in dem Öl und/oder in dem Kraftstoff und/oder in der Hydraulikflüssigkeit,
- einen relativen und/oder absoluten Feuchtegehalt und/oder
- weitere physikalisch/chemisch relevante Parameter
des Kraftstoffs und/oder des Öls und/oder der Hydraulikflüssigkeit umfasst.

Aussagen zu den Bindungszuständen in dem Öl und/oder Kraftstoff und/oder in der Hydraulikflüssigkeit erlauben festzustellen, um welches Material bzw. welchen Bestandteil des Öls und/oder des Kraftstoffs und/oder der Hydraulikflüssigkeit es sich handelt. Beispielsweise lassen sich auf diese Weise unterschiedliche Bestandteile, Verunreinigungen, Einschlüsse oder dergleichen detektieren und unterscheiden. Ebenfalls kann eine Bestimmung einer Materialkonzentration in dem untersuchten Öl und/oder Kraftstoff und/oder der zu untersuchenden Hydraulikflüssigkeit realisiert werden, sofern eine Kalibrierung des Kernspinresonanz-Sensors vor der Messung erfolgt. In einer Ausführungsform kann diese Materialkonzentration mit einem zulässigen Grenzwert (Schwellenwert) verglichen werden und bei Überschreiten des Grenzwerts eine Warnung durch das Messgerät ausgegeben werden.

Mit der Aufnahme und Auswertung zeitlich-dynamischer Prozesse chemischer Verbindungen können Prozesse wie Diffusion von Feuchtigkeit und/oder Zersetzung in einem Öl und/oder Kraftstoff und/oder in einer Hydraulikflüssigkeit untersucht werden. Rückschlüsse auf eine mögliche Alterung des Kraftstoffs und/oder des Öls und/oder der Hydraulikflüssigkeit lassen sich daraus ableiten. Darüber hinaus kann das Messgerät ebenfalls eingesetzt werden, ein Öl und/oder Kraftstoff und/oder eine Hydraulikflüssigkeit hinsichtlich Feuchtigkeit umfassend zu charakterisieren, beispielsweise Aussagen über den relativen und/oder absoluten Feuchtegehalt sowie über einen Feuchtegradienten in das Öl und/oder in den Kraftstoff und/oder in die Hydraulikflüssigkeit hinein zu ermöglichen.

Weitere physikalisch und/oder chemisch relevante Parameter, die unter Verwendung des Messgeräts mit der Auswertevorrichtung ausgewertet werden können, umfassen insbesondere eine Dichte, eine Porosität, eine Viskosität oder dergleichen des untersuchten Kraftstoffs und/oder Öls und/oder der untersuchten Hydraulikflüssigkeit.

Je nach gewünschter Information misst der Kernspinresonanz-Sensor ein Spektrum und/oder Relaxationskurven und/oder Relaxationszeiten, wobei die Auswertevorrichtung die gezielte Auswertung dieser Messsignale hinsichtlich der gewünschten Information durchführt.

In einer Ausgestaltung wird das Messgerät zur Untersuchung eines Kraftstoffs und/oder Öls und/oder einer Hydraulikflüssigkeit verwendet, wobei mittels der Auswertevorrichtung des Messgeräts eine ermittelte Information, insbesondere ein Spektrum und/oder eine Relaxationszeit, mit Referenzdaten einer Referenzdatenbank verglichen wird.

Auf diese Weise kann eine besonders einfache und umfassende Auswertung und/oder Bewertung und/oder Interpretation eines gemessenen Messsignals des Kernspinresonanz-Sensors erfolgen. Beispielsweise kann ein aufgenommenes Spektrum und/oder aufgenommene Relaxationskurven, insbesondere Relaxationszeiten, mit Referenzspektren, Referenzrelaxationskurven bzw. Referenzrelaxationszeiten verglichen werden. Der Vergleich der Messsignale mit im Detail bestimmten und bekannten Referenzdaten dient dabei der schnellen und einfachen Zuordnung des Messsignals, um eine bestimmte Information aus dem gemessenen Messsignal abzuleiten. Durch den Vergleich mit bekannten Referenzdaten kann ferner - neben einer sehr hohen Auswertegeschwindigkeit - ein besonders genaues Ergebnis, d.h. eine besonderes genaue Information über das untersuchte Öl und/oder den untersuchten Kraftstoff und/oder die untersuchte Hydraulikflüssigkeit erhalten werden. Beispielsweise kann in einem Vergleich mit einem Referenzspektrum eine Verschiebung eines gemessenen Spektrums sehr leicht und schnell erkannt werden, sodass aus dieser Verschiebung eine Information betreffend das untersuchte Öl und/oder den Kraftstoff und/oder die untersuchte Hydraulikflüssigkeit ableitbar ist.

Vorteilhaft können durch den Vergleich der gemessenen Messsignale mit Referenzdaten auf besonders einfache Weise Abweichungen der Messsignale von den Referenzdaten identifiziert werden. Übersteigen diese Abweichungen eine definierte Toleranzschwelle, können sie als Indiz für Unregelmäßigkeit und/oder Unstimmigkeiten betreffend das untersuchte Öl und/oder den untersuchten Kraftstoff und/oder die untersuchte Hydraulikflüssigkeit interpretiert werden.

Die Referenzdaten, insbesondere Referenzkurven und/oder Referenzwerte und/oder Referenzspektren, können dabei geräteintern in einer Datenbank auf einer Speichereinheit, insbesondere einer Speichereinheit der Steuer- und/oder der Auswertevorrichtung, gespeichert sein. In einer alternativen oder zusätzlichen Ausführungsform können die Referenzdaten auch in einer geräteexternen, vorteilhaft stets aktuellen, Referenzdatenbank gespeichert sein, beispielsweise in einer Referenzdatenbank auf einem Computer, einem Server oder auf einem anderen, einem Fachmann sinnvoll erscheinenden Datenspeicher und/oder Datenverarbeitungsgerät. Insbesondere kann der Vergleich der gemessenen Messsignale mit den Referenzdaten über einen Internetzugang des Messgeräts erfolgen. Alternativ oder zusätzlich können geräteintern gespeicherte Referenzdaten ebenfalls über einen Internetzugang des Messgeräts aktualisiert werden, beispielsweise durch einen Abgleich mit einer geräteexternen Referenzdatenbank.

In einer Ausgestaltung wird das Messgerät zur Untersuchung eines Kraftstoffs und/oder Öls und/oder einer Hydraulikflüssigkeit verwendet, wobei mittels der Ausgabevorrichtung des Messgeräts, insbesondere mittels eines Displays, eine ermittelte Information, insbesondere ein Spektrum und/oder eine Relaxationszeit, und/oder eine Abweichung einer ermittelten Information von Referenzdaten einer Referenzdatenbank, ausgegeben wird, insbesondere dargestellt wird.

Unter Verwendung der mittels der Ausgabevorrichtung ausgegebenen, insbesondere dargestellten, Information ist es möglich, insbesondere einem Bediener des Messgeräts möglich, nach Durchführung der Untersuchung des Kraftstoffs und/oder Öls und/oder der Hydraulikflüssigkeit zu einem Ergebnis zu gelangen.

In einer Ausführungsform werden die das untersuchte Öl und/oder den untersuchten Kraftstoff betreffenden Informationen in einer intuitiv verständlichen, insbesondere aufbereiteten Weise dem Bediener des Messgeräts mittels eines Displays der Ausgabevorrichtung ausgegeben. Intuitiv ist die Ausgabe insbesondere dann, wenn der Bediener ohne Vorkenntnisse eine Untersuchung eines Kraftstoffs und/oder eines Öls und/oder einer Hydraulikflüssigkeit durchführen kann und anschließend eine Aussage, bevorzugt eine Bewertung, aus den angezeigten Informationen ableiten kann. In einer Ausführungsform kann eine Aufbereitung der ermittelten Information beispielsweise in Form einer Farbzuordnung erfolgen. Dabei kann beispielsweise Rot eine ermittelte bedenkliche Abweichung einer untersuchten Zielgröße - beispielsweise Qualität - von einer Vorgabe - beispielsweise definiert durch die Referenzdaten in der Referenzdatenbank - signalisieren. Gelb hingegen signalisiert eine Abweichung innerhalb einer zulässigen Toleranz und Grün eine ermittelte Abweichung in zulässigem und/oder unbedenklichem Maße. Alternativ oder zusätzlich kann eine Ausgabe der ermittelten Information in Form einer Kurznachricht erfolgen, die auf dem Display dargestellt wird. Diese Kurznachricht kann beispielsweise eine Spezifikation der zur Bewertung des untersuchten Kraftstoffs und/oder Öls und/oder der Hydraulikflüssigkeit herangezogenen Referenzdaten beinhalten, ferner eine Bewertung der Abweichung, sofern ermittelt, sowie daraus abgeleitete Empfehlungen wie "Öl gealtert, Austausch empfohlen" oder "Dichte und Viskosität entsprechen Herstellerangaben" oder dergleichen.

In einer alternativen oder zusätzlichen Ausgestaltung wird das Messgerät zur Untersuchung eines Kraftstoffs und/oder Öls und/oder einer Hydraulikflüssigkeit verwendet, wobei eine ermittelte Information, insbesondere ein Spektrum und/oder eine Relaxationszeit, und/oder eine Abweichung einer ermittelten Information von Referenzdaten einer Referenzdatenbank, an und/oder in einem Fahrerstand ausgegeben werden/wird, insbesondere dargestellt werden/wird. Auf diese Weise kann eine mittels eines in einem Fahrzeug integrierten Messgeräts ermittelte Information während des Betriebs des Fahrzeug an den Fahrzeugführer ausgegeben werden.

Das Messgerät wird zur Untersuchung eines Kraftstoffs und/oder Öls und/oder einer Hydraulikflüssigkeit verwendet, wobei eine ermittelte Information zur Steuerung und/oder Regelung zumindest einer Komponente des Fahrzeugs verwendet wird. Die das untersuchte Öl und/oder den untersuchten Kraftstoff und/oder die untersuchte Hydraulikflüssigkeit betreffenden Informationen werden an ein weiteres externes Gerät, insbesondere an eine Komponente eines Fahrzeugs, beispielsweise eine Motorsteuerung, eine Bremskraftverstärkersteuerung oder dergleichen, weitergeleitet. Auf diese Weise dienen die Informationen der verbesserten, insbesondere optimierten Steuerung und/oder Regelung des externen Geräts bzw. der Komponente des Fahrzeugs oder des mittels des externen Geräts bzw. der Komponente des Fahrzeugs gesteuerten und/oder geregelten Systeme - beispielsweise eines Motors. In einem konkreten Ausführungsbeispiel kann unter Verwendung des Messgeräts insbesondere eine Dichte, Porosität, Viskosität, ein Wasser- sowie ein Gasgehalt einer Bremsflüssigkeit eines Kraftfahrzeugs während des Betriebs des Kraftfahrzeugs laufend oder in regelmäßigen Abständen bestimmt werden. Die auf diese Weise ermittelten Informationen werden anschließend an eine Bremskraftverstärkersteuerung des Kraftfahrzeugs weitergeleitet und von dieser zur optimierten Steuerung und/oder Regelung der Bremskraft verwendet. So kann beispielsweise bei einem relativ hohen Gasgehalt in der Bremsflüssigkeit eine stärkere Bremskraftverstärkung während eines Bremsvorganges gewählt werden, die die Kompressibilität des in der Bremsflüssigkeit enthaltenen Gases ausgleicht.

In einer Ausgestaltung wird das Messgerät zur Untersuchung eines Kraftstoffs und/oder Öls und/oder einer Hydraulikflüssigkeit verwendet, wobei mittels einer Eingabevorrichtung Angaben zu einem Öl und/oder Kraftstoff und/oder einer Hydraulikflüssigkeit durch Bedienereingaben spezifiziert und dem Messgerät zur Verfügung gestellt werden.

Unter einer Eingabevorrichtung soll insbesondere ein Mittel verstanden werden, das dazu vorgesehen ist, zumindest eine Information von einem Bediener des Messgeräts über eine akustische, optische, gestengestützte und/oder taktile Eingabe anzunehmen und an die Steuervorrichtung des Messgeräts weiterzuleiten. Beispielsweise kann die Eingabevorrichtung aus einem Betätigungselement, einer Tastatur, einem Display, insbesondere einem Touch-Display, einem Spracheingabemodul, einer Gestenerkennungseinheit und/oder einem Zeigegerät (beispielsweise Maus) bestehen. Alternativ oder zusätzlich kann die Eingabevorrichtung auch außerhalb des Messgeräts realisiert sein, beispielsweise in Form eines externen Datenverarbeitungsgeräts wie einem Smartphone, einem Tablet-PC, einem PC, oder einem Bordcomputers eines Fahrzeugs oder dergleichen, das über eine Datenkommunikationsschnittstelle mit der Steuervorrichtung des Messgeräts verbunden ist.

Durch die Eingabe von Angaben zu einem Öl und/oder Kraftstoff und/oder einer Hydraulikflüssigkeit lassen sich eine Informationsverarbeitung, insbesondere die Auswertung des Messsignals, der Vergleich eines Messsignals und/oder einer ermittelten Information mit Referenzdaten oder dergleichen, vorteilhaft an das zu untersuchende Öl und/oder den Kraftstoff und/oder die Hydraulikflüssigkeit anpassen. Beispielsweise kann in Abhängigkeit einer Bedienereingabe eine Referenzdatenbank gewählt werden. Ferner können insbesondere im Zusammenhang mit der Spezifikation ein Betriebsprogramm der Steuervorrichtung, Regelroutinen, Steuerroutinen, Auswerteroutinen und/oder Berechnungsroutinen angepasst werden.

"Angaben zu einem Öl und/oder Kraftstoff und/oder einer Hydraulikflüssigkeit" können beispielsweise das Öl und/oder den Kraftstoff und/oder die Hydraulikflüssigkeit selbst charakterisieren ("Diesel", "Benzin", "Biodiesel", "Bremsflüssigkeit") und/oder einen Hersteller betreffen. Alternativ oder zusätzlich können weitere, insbesondere die physikalischen und/oder chemischen Eigenschaften des Kraftstoffs und/oder Öls und/oder der Hydraulikflüssigkeit betreffende Angaben sinnvoll und/oder notwendig sein.

In einer Ausgestaltung wird das Messgerät zur Untersuchung eines Kraftstoffs und/oder Öls und/oder einer Hydraulikflüssigkeit verwendet, wobei vor Untersuchung eines Kraftstoffs und/oder Öls und/oder einer Hydraulikflüssigkeit unter Verwendung einer geräteintern vorgesehenen Standardprobe, insbesondere unter Verwendung einer geräteintern vorgesehenen Tetramethylsilan-Probe, eine Kalibrierung des Messgeräts durchführt wird.

Zur genaueren Untersuchung eines Kraftstoffs und/oder Öls und/oder einer Hydraulikflüssigkeit kann somit vor Durchführung der Untersuchung eine Kalibrierung des Messgeräts, insbesondere eine Kalibrierung des Kernspinresonanz-Sensors, erfolgen. Die Kalibrierung erfolgt in einer Ausführungsform unter Verwendung einer reinen Materialprobe, bevorzugt unter Verwendung einer, insbesondere geräteintern vorgesehenen, Tetramethylsilan-Probe (TMS), die als Standard verwendet wird. Alle im Anschluss an die Kalibrierung folgenden Messungen, insbesondere Messungen an einem zu untersuchenden Öl und/oder Kraftstoff und/oder an einer zu untersuchenden Hydraulikflüssigkeit, werden in Bezug zu dieser Kalibriermessung ausgewertet.

Des Weiteren wird ein Messgerät, insbesondere handgehaltenes Messgerät, bevorzugt ein Messgerät umfassend ein Gehäuse, zur Untersuchung von Öl und/oder Kraftstoff und/oder Hydraulikflüssigkeit vorgeschlagen, mit zumindest
- einem Kernspinresonanz-Sensor,
- einer Steuervorrichtung zur Steuerung des Messgeräts,
- einer Auswertevorrichtung zur Auswertung eines von dem Kernspinresonanz-Sensor gelieferten Messsignals,
- einer Ausgabevorrichtung zur Ausgabe von ermittelten Informationen sowie
- einer Energieversorgungsvorrichtung,
wobei das Messgerät, insbesondere der Kernspinresonanz-Sensor und/oder die Auswertevorrichtung, zur Untersuchung von Öl und/oder Kraftstoff und/oder Hydraulikflüssigkeit vorgesehen ist. Das Messgerät wird erfindungsgemäss nach Anspruch 1 verwendet.

Die bereits ausgeführten Beschreibungen bezüglich der Verwendung des Messgeräts, insbesondere die Ausführungen zur Auswertevorrichtung und zum Kernspinresonanz-Sensor, gelten entsprechend natürlich auch für das Messgerät selbst.

In einer Ausführungsform des Messgeräts ist ferner eine Speichervorrichtung zum Speichern von Messergebnissen und/oder Arbeitsparametern vorgesehen. Diese Speichervorrichtung kann alle Formen an externen und internen elektronischen, insbesondere digitalen Speichern, umfassen, insbesondere auch Speicherchips wie USB-Sticks, Memory-Sticks, Speicherkarten, etc.

Darüber hinaus wird vorgeschlagen, dass die Steuervorrichtung und/oder die Auswertevorrichtung des erfindungsgemäßen Messgeräts eine Datenkommunikationsschnittstelle zur, insbesondere drahtlosen, Kommunikation aufweist, mittels der das Messgerät Messergebnisse und/oder Arbeitsparameter senden und/oder empfangen kann. Bevorzugt verwendet die Datenkommunikationsschnittstelle ein standardisiertes Kommunikationsprotokoll zu einer Übertragung von elektronischen, insbesondere digitalen Daten. Vorteilhaft umfasst die Datenkommunikationsschnittstelle eine drahtlose Schnittstelle, insbesondere beispielsweise eine WLAN-, Bluetooth-, Infrarot-, NFC-, RFID-, GSM-Schnittstelle oder eine andere, einem Fachmann als sinnvoll erscheinende drahtlose Schnittstelle. Alternativ kann die Datenkommunikationsschnittstelle auch einen kabelgebunden Adapter aufweisen, beispielsweise einen USB- oder Mikro-USB-Adapter. Vorteilhaft können mittels der Datenkommunikationsschnittstelle Messergebnisse und/oder Arbeitsparameter von dem Messgerät an ein externes Datengerät, beispielsweise an ein Smartphone, einen Tablet-PC, einen PC, einen Drucker oder weitere einem Fachmann als sinnvoll erscheinende externe Geräte gesendet werden oder von diesen empfangen werden. Mittels der erfindungsgemäßen Ausgestaltung kann vorteilhaft eine Übertragung von Referenzdaten ermöglicht werden, die zu einer weiteren Auswertung von mit dem Messgerät erfassten Messsignalen nutzbar ist. Insbesondere werden die Referenzdaten dabei von einer geräteinternen Referenzdatenbank abgerufen. Ferner können vorteilhaft vielfältige Zusatzfunktionen ermöglicht und eingebunden werden, die insbesondere auch eine direkte Kommunikation mit Smartphones (insbesondere über programmierte Apps) oder ähnlichen portablen Datengeräten erfordern. Diese können beispielsweise automatische Kartierungs-Funktionen, Firmware-Updates, Datennachbearbeitung, Datenaufbereitung, Datenabgleich mit anderen Geräten, etc. umfassen.

Ferner wird ein Verfahren zur Untersuchung von Öl und/oder Kraftstoff und/oder Hydraulikflüssigkeit mittels eines Messgeräts, insbesondere eines handgehaltenen Messgeräts, vorgeschlagen, welches nicht unter den Schutzumfang der Erfindung fällt. Das Verfahren ist insbesondere durch zumindest folgende Schritte gekennzeichnet.
i. Kalibrieren des Kernspinresonanz-Sensors unter Verwendung einer Standardprobe, insbesondere unter Verwendung einer geräteintern vorgesehenen Tetramethylsilan- Probe
ii. Messung zumindest eines Spektrums und/oder einer Relaxationszeit resultierend aus der Anregung von Kernspins in dem zu untersuchenden Öl und/oder in dem zu untersuchenden Kraftstoff und/oder in der zu untersuchenden Hydraulikflüssigkeit
iii. Auswertung von Messsignalen des Kernspinresonanz-Sensors durch Vergleich der Messsignale mit Referenzdaten einer Referenzdatenbank
iv. Ausgabe der Auswerteergebnisse, insbesondere einer ermittelten Information und/oder einer Abweichung einer ermittelten Information von Referenzdaten einer Referenzdatenbank.

### Zeichnungen

Die Erfindung ist anhand von in den Zeichnungen dargestellten Ausführungsbeispielen in der nachfolgenden Beschreibung näher erläutert. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Gleiche oder ähnliche Bezugszeichen in den Figuren bezeichnen gleiche oder ähnliche Elemente.

Es zeigen:
- Figur 1: eine perspektivische Darstellung einer Ausgestaltung des erfindungsgemäßen Messgeräts,
- Figur 2: eine Ansicht der ersten Gehäuseseite einer Ausgestaltung des erfindungsgemäßen Messgeräts,
- Figur 3: eine schematische Seitenansicht einer Ausgestaltung des erfindungsgemäßen Messgeräts,
- Figur 4a: eine schematische Schnittdarstellung einer Ausführungsform der den Kernspinresonanz-Sensor bildenden Komponenten sowie der damit erzeugten Magnetfelder,
- Figur 4b: eine schematische Schnittdarstellung einer alternativen Ausführungsform der den Kernspinresonanz-Sensor bildenden Komponenten sowie der damit erzeugten Magnetfelder,
- Figur 5a: ein perspektivische Darstellung einer alternativen Ausgestaltung des erfindungsgemäßen Messgeräts,
- Figur 5b: eine schematische Schnittdarstellung einer alternativen Ausführungsform der den Kernspinresonanz-Sensor bildenden Komponenten sowie der damit erzeugten Magnetfelder,
- Figur 5c: eine schematische Schnittdarstellung einer alternativen Ausführungsform der den Kernspinresonanz-Sensor bildenden Komponenten sowie der damit erzeugten Magnetfelder,
- Figur 6: ein Verfahrensdiagramm einer Ausführungsform des erfindungsgemäßen Verfahrens.

### Beschreibung der Ausführungsbeispiele

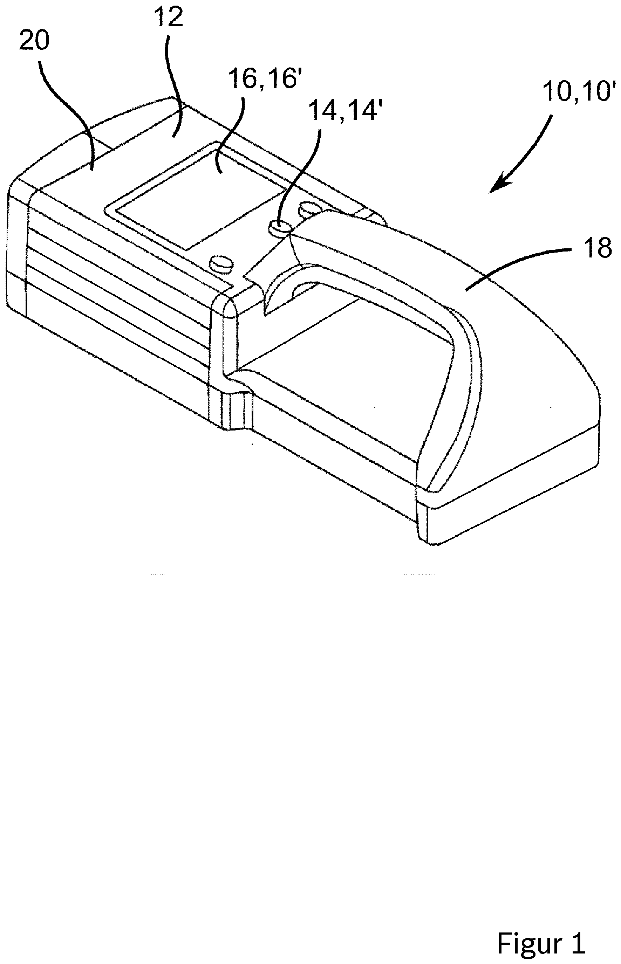

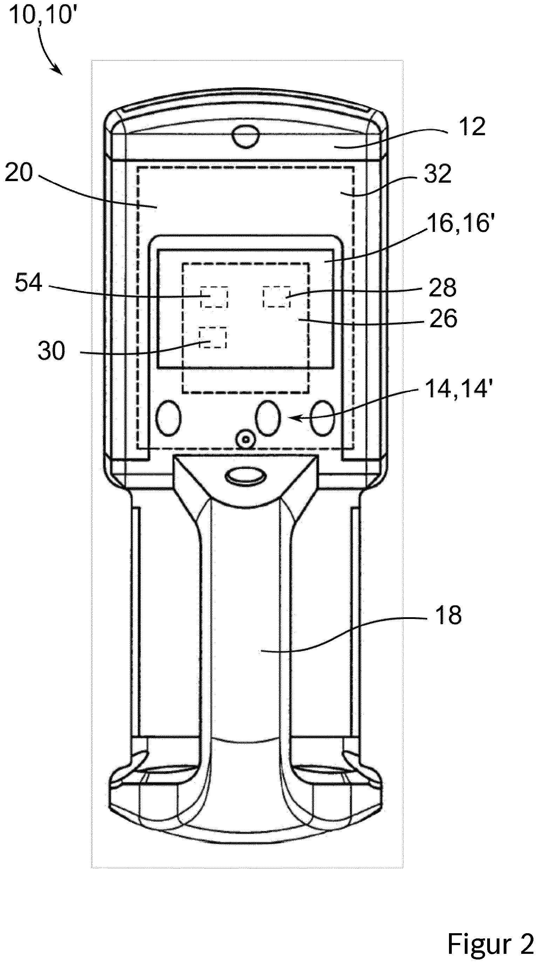

Figur 1 und Figur 2 zeigen zwei Ansichten einer beispielhaften Ausführungsform des Messgeräts 10 in perspektivischer Darstellung bzw. in vereinfachter, schematischer Aufsicht. Das Messgerät 10 ist in der dargestellten Ausführungsform als ein handgehaltenes, energieautonomes Messgerät 10' realisiert.

Das beispielhaft ausgeführte Messgerät 10' weist ein Gehäuse 12 auf. In dem Gehäuse 12 ist eine Eingabevorrichtung 14 in Form von Betätigungselementen 14', geeignet zum Ein- und Ausschalten des Messgeräts 10', zum Starten und Konfigurieren eines Messvorgangs und zum Eingeben von Arbeitsparametern, untergebracht. Ferner ist in dem Gehäuse 12 eine Ausgabevorrichtung 16 zur Ausgabe von ermittelten Informationen sowie zur Ausgabe von Arbeitsparametern in Form eines Displays 16' vorgesehen. Das Messgerät 10' verfügt zum Transport und zu dessen Führung über einen Handgriff 18. Der Handgriff 18, die Betätigungselemente 14' sowie das Display 16' befinden sich auf einer ersten Gehäuseseite 20 des Messgeräts 10 (auch "Frontseite"), die bei einer Bedienung des Messgeräts typischerweise dem Bediener zugewandt ist.

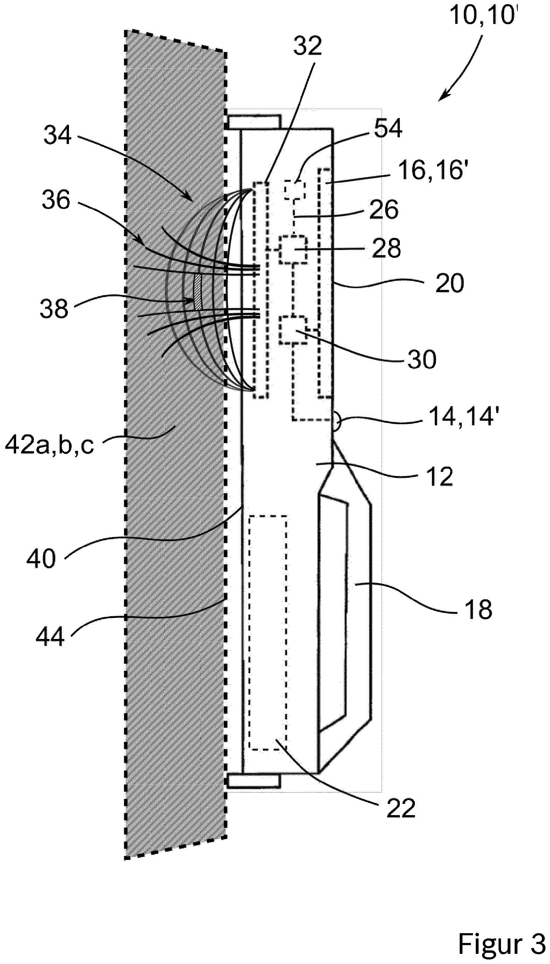

Zur Energieversorgung des Messgerät 10' weist das Messgerät 10' auf der, der ersten Gehäuseseite 20 geräterückseitig gegenüberliegenden, zweiten Gehäuseseite 40 (im Folgenden auch Rückseite des Messgeräts genannt) eine Aussparung auf, die der Aufnahme von stromnetzunabhängigen Energiespeichern 22 in Form von wiederaufladbaren Akkus dient (vgl. Figur 3). Auf Grund des stromnetzunabhängigen Energiespeichers 22 kann das Messgerät 10' zumindest vorübergehend energieautonom, d.h. unabhängig von einem Stromnetz und somit insbesondere auch kabellos, betrieben werden kann. Das beispielhaft vorgestellte Messgerät 10' besitzt Lithium-Ionen-Akkus, deren hohe Energie- und Leistungsdichte vorteilhaft zur Energieversorgung des Messgeräts 10' geeignet ist. In einer alternativen Ausführungsform kann der Energiespeicher 22 auch im Handgriff 18 des Messgeräts 10' untergebracht sein. Vorzugsweise weist die Energieversorgungsvorrichtung eine lösbare Formschluss- und/oder Kraftschlussverbindungsschnittstelle auf, sodass der Energiespeicher 22 (im Allgemeinen auch mehrere) abnehmbar und austauschbar anordenbar ist. Darüber hinaus lässt sich der Energiespeicher 22 in und/oder außerhalb des Messgeräts mit Energie aus einem Stromnetz versorgen und laden.

Auf einem Trägerelement 26, insbesondere einer Systemplatine oder Leiterplatte innerhalb des Gehäuses 12, sind weitere Komponenten des Messgeräts 10', insbesondere ein Kernspinresonanz-Sensor 32, eine Steuervorrichtung 28 zur Steuerung des Messgeräts 10', eine Auswertevorrichtung 30 zur Auswertung von von dem Kernspinresonanz-Sensor 32 gelieferten Messsignalen, sowie eine mit der Steuer- und/oder Auswertevorrichtung verbundene Datenkommunikationsschnittstelle 54, untergebracht (siehe insbesondere Figur 2 und 3).

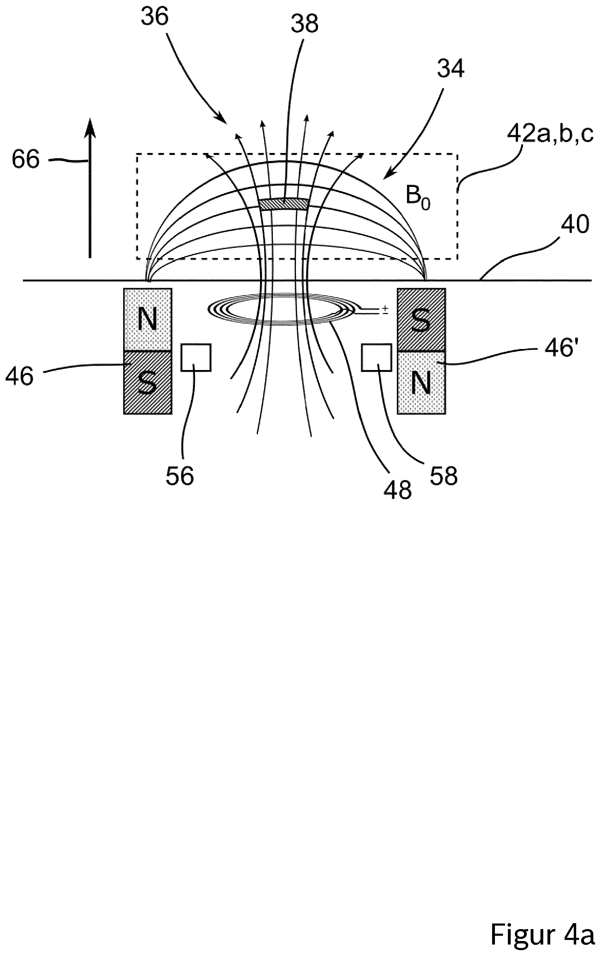

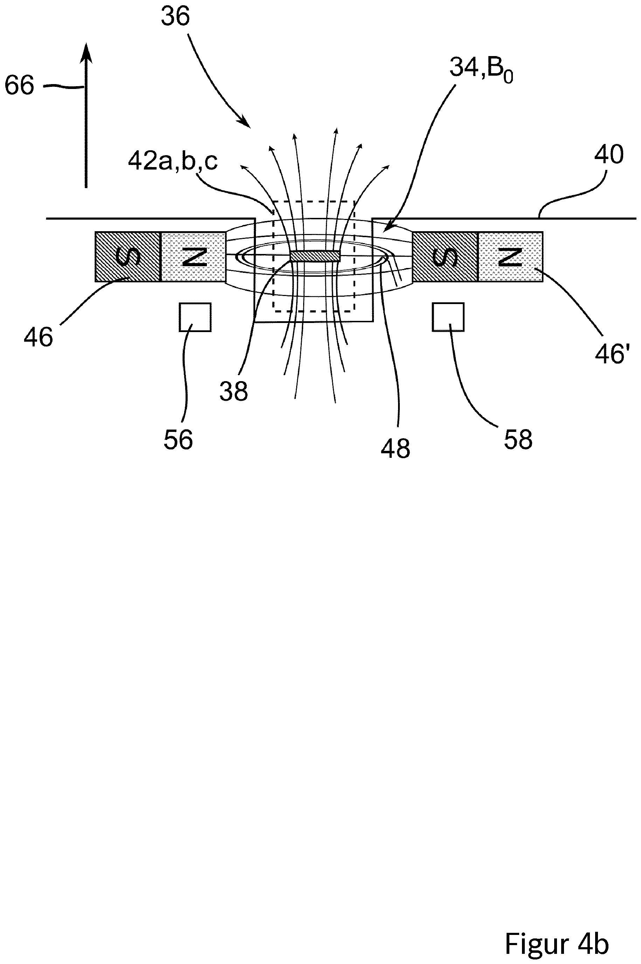

Der Kernspinresonanz-Sensor 32, der im Detail in der Beschreibung zu den Figuren 4a und 4b erläutert wird, ist zur Anregung einer Kernspinresonanz in Atomkernen des Materials des Kraftstoffs 42a und/oder Öls 42b und/oder der Hydraulikflüssigkeit 42c vorgesehen. Das gemessene Resonanzsignal, insbesondere ein Spektrum, Relaxationskurven und/oder Relaxationszeiten, wird zumindest zur zerstörungs- und kontaminationsfreien Untersuchung des Kraftstoffs 42a und/oder Öls 42b und/oder der Hydraulikflüssigkeit 42c verwendet. Auf diese Weise können Informationen ermittelt werden, die unter anderem die Qualität, Alter, Reinheit, Zusammensetzung, Viskosität oder dergleichen des Kraftstoffs 42a und/oder des Öls 42b und/oder der Hydraulikflüssigkeit 42c betreffen.

Die Steuervorrichtung 28 weist eine Steuerelektronik umfassend Mittel zur Kommunikation mit den anderen Komponenten des Messgeräts 10' auf, beispielsweise Mittel zur Steuerung und Regelung des Kernspinresonanz-Sensors 32, der Auswertevorrichtung 30 und dergleichen. Die Steuervorrichtung 28 umfasst insbesondere eine Einheit mit einer Prozessoreinheit, einer Speichereinheit und einem in der Speichereinheit gespeicherten Betriebsprogramm. Die Steuervorrichtung 28 ist dazu vorgesehen, zumindest ein Betriebsfunktionsparameter des Messgeräts 10' in Abhängigkeit von zumindest einer Eingabe durch den Bediener, durch die Auswertevorrichtung 30 und/oder durch die Datenkommunikationsschnittstelle 54 einzustellen.

Die Auswertevorrichtung 30 zur Auswertung von von dem Kernspinresonanz-Sensor 32 gelieferten Messsignalen weist insbesondere einen Informationseingang, eine Informationsverarbeitung und einen Informationsausgang auf (nicht näher dargestellt). Vorteilhaft besteht die Auswertevorrichtung 30 zumindest aus einem Prozessor, einem Speicher mit einem darauf gespeicherten und ausführbaren Betriebsprogramm und erlaubt, zumindest ein Messsignal des Kernspinresonanz-Sensors 32 auszuwerten und somit Informationen betreffend die Qualität, Alter, Reinheit, Zusammensetzung oder dergleichen des Kraftstoffs 42a und/oder Öls 42b und/oder der Hydraulikflüssigkeit 42c zu bestimmen. Ferner weist die Auswertevorrichtung 30 gespeicherte Korrektur- und/oder Kalibriertabellen auf, die es erlauben, die Auswerteergebnisse zu interpretieren, umzurechnen, zu inter- und/oder extrapolieren sowie das Messgerät 10', insbesondere die Auswerteroutinen, hinsichtlich eines Kraftstoffs 42a und/oder Öls 42b und/oder einer Hydraulikflüssigkeit 42c zu kalibrieren. Die Auswerteergebnisse werden von der Auswertevorrichtung 30 zur weiteren Verwendung über die Steuervorrichtung 28 entweder direkt an einen Bediener des Messgeräts 10' oder zur Versendung der Daten an die Datenkommunikationsschnittstelle 54 ausgegeben. Insbesondere können die Auswerteergebnisse und/oder Messsignale unter Verwendung der Datenkommunikationsschnittstelle 54 mit in einer Referenzdatenbank gespeicherten Referenzdaten verglichen werden.

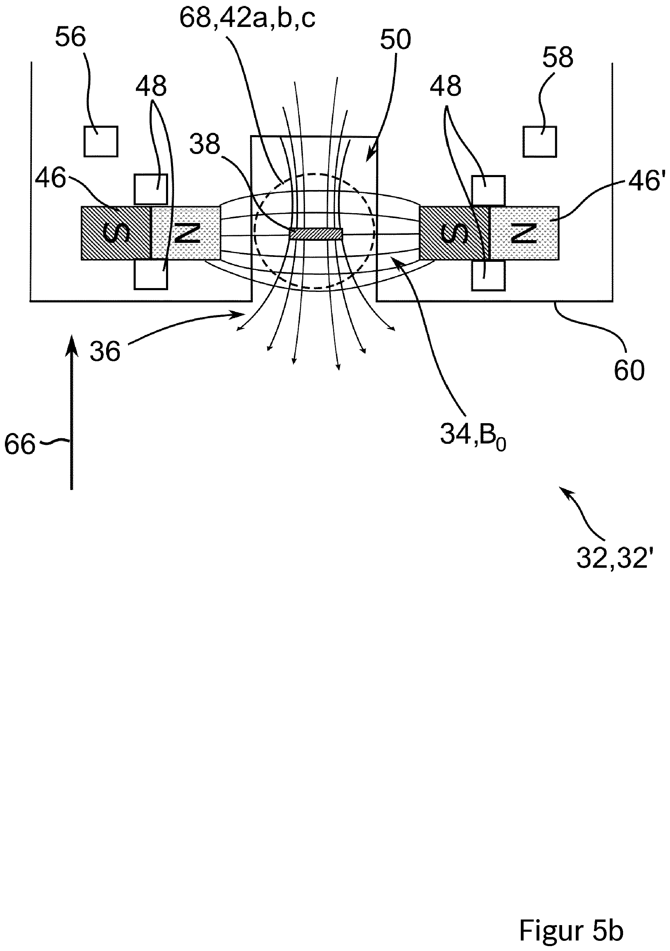

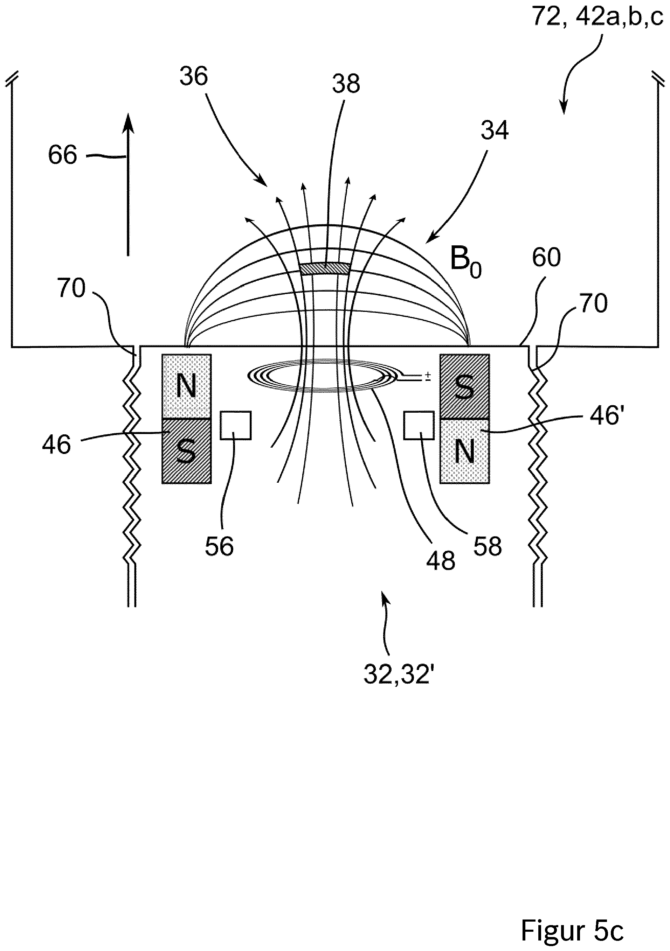

In Figur 3 ist eine Ausführungsform eines Messgeräts 10', insbesondere des handgehaltenen Messgeräts 10' der Figuren 1 und 2, in einer vereinfachten schematischen Seitenansicht dargestellt. Der Kernspinresonanz-Sensor 32 umfasst zwei Vorrichtungen zur Erzeugung von Magnetfeldern, insbesondere eine Permanentmagnetanordnung 46,46' (vgl. Figur 4a, 4b, aber auch Figur 5b und 5c), die ein erstes Magnetfeld 34 erzeugt (B₀), sowie eine Hochfrequenzspule 48 (vgl. Figuren 4a, 4b, 5b, 5c), die ein zweites Magnetfeld 36 erzeugt. Der Kernspinresonanz-Sensor 32 ist derart konfiguriert, dass das erste Magnetfeld 34 (in den Figuren durch Magnetfeldlinien repräsentiert) im Wesentlichen parallel zu der zweiten Gehäuseseite 40 ausgerichtet ist, während das zweite Magnetfeld 36 im Wesentlichen senkrecht zu den Magnetfeldlinien des ersten Magnetfeldes 34 ausgerichtet ist. Die beiden Magnetfelder überlagern sich in einem ausgedehnten Bereich, in dem sich der sensitive Bereich 38 des Kernspinresonanz-Sensors 32 als insbesondere schichtförmiger Bereich befindet. Das handgehaltene Messgerät 10' wird mit der zweiten Gehäuseseite 40 in unmittelbarer Nähe an ein zu untersuchenden Kraftstoff 42a und/oder an ein zu untersuchendes Öl 42b und/oder an eine zu untersuchende Hydraulikflüssigkeit 42c derart positioniert, dass der Abstand zwischen der zweiten Gehäuseseite 40 und der Oberfläche 44 des Kraftstoffs 42a und/oder Öls 42b und/oder der Hydraulikflüssigkeit 42c (Flüssigkeitsoberfläche oder alternativ auch Behältnisoberfläche) minimiert ist. Auf diese Weise wird erreicht, dass die Magnetfelder 34,36 in das Öl 42b und/oder in den Kraftstoff 42a und/oder in die Hydraulikflüssigkeit 42c eindringen und der sensitive Bereich 38 in dem Öl 42b und/oder in dem Kraftstoff 42a und/oder in der Hydraulikflüssigkeit 42c zu liegen kommt.

Durch Variation des durch die zweite Vorrichtung erzeugten zweiten Magnetfeldes 36, d.h., insbesondere durch Variation der Hochfrequenzspule 48 und/oder Variation der Frequenz und/oder Variation des Stroms und/oder Variation der Spannung in der Hochfrequenzspule 48, ist es möglich, den sensitiven Bereich 38 in seinem Abstand zu der zweiten Gehäuseseite 40 (in Richtung 66 (vgl. insbesondere Figuren 4a, 4b, 5b, 5c) in das Öl 42b und/oder in den Kraftstoff 42a und/oder in die Hydraulikflüssigkeit 42c hinein) zu verändern und somit den Abstand des sensitiven Bereichs 38 im Öl 42b und/oder im Kraftstoff 42a und/oder in der Hydraulikflüssigkeit 42c zu modifizieren. Alternativ und/oder zusätzlich kann der Kernspinresonanz-Sensor 32 im Gehäuse 12 des Messgeräts 10' derart umpositioniert werden, dass der Abstand des Kernspinresonanz-Sensors 32 zur zweiten Gehäuseseite 40 verändert wird und folglich auch der Abstand des sensitiven Bereichs 38 im Öl 42a und/oder im Kraftstoff 42a und/oder in der Hydraulikflüssigkeit 42c zu dessen/deren Oberfläche 44. Besonders vorteilhaft lassen sich auf diese Weise Tiefenprofile der auszuwertenden Informationen erstellen. Beispielsweise ist es möglich, über ein Tiefenprofil eines Feuchteverlaufs in einem Öl 42b und/oder Kraftstoff 42 42a eine Aussage über den Fortschritt eines Alterungsprozesses zu treffen.

In Figur 4a sind in schematischer Schnittdarstellung eines Details eines Ausführungsbeispiels eines Messgeräts 10, insbesondere des handgehaltenen Messgeräts 10', der Kernspinresonanz-Sensor 32 zusammen mit einem zu untersuchenden Öl 42b und/oder Kraftstoff 42a und/oder einer zu untersuchenden Hydraulikflüssigkeit 42c dargestellt. Zwei senkrecht zur zweiten Gehäuseseite 40 des Messgeräts 10' und antiparallel zueinander angeordnete Permanentmagnete 46,46' erzeugen ein erstes, insbesondere statisches, Magnetfeld 34, das im Wesentlichen parallel zur Oberfläche der zweiten Gehäuseseite 40 verläuft. Dieses zur Ausrichtung der Kernspins der in dem Öl 42b und/oder in dem Kraftstoff 42a und/oder in der Hydraulikflüssigkeit 42c vorhandenen Atomkerne vorgesehene erste Magnetfeld 34 weist beispielhaft insbesondere eine Magnetfeldstärke von 0.5 Tesla auf, wobei die Permanentmagnete 46,46' aus einer Neodym-Eisen-Bor-Legierung hergestellt sind. In einer alternativen Ausführungsform kann das Magnetfeld 34 auch mittels eines Elektromagneten erzeugt werden. Die zweite Vorrichtung zur Erzeugung des zweiten Magnetfeldes wird in diesem Ausführungsbeispiel durch eine Hochfrequenzspule 48 gebildet. Sobald durch diese Spule Strom fließt, wird ein elektromagnetisches Feld, insbesondere das zweite Magnetfeld 36, induziert. Die beiden Magnetfelder 34,36 überlagern sich in einem Bereich, der im Wesentlichen außerhalb des Gehäuses 12 des Messgeräts 10' liegt. Der sensitive Bereich 38 des Kernspinresonanz-Sensors 32 liegt ebenfalls in dem Überlagerungsfeld der Magnetfelder 34 und 36. In Abhängigkeit der Frequenz des eingestrahlten elektromagnetischen Felds 36 und der statischen Magnetfeldstärke des ersten Magnetfelds 34 wird der sensitive Bereich im Idealfall durch eine Fläche definiert, auf der die Magnetfeldstärke des ersten Magnetfelds 34 konstant ist und insbesondere einen definierten Betrag aufweist. In Realität ist die Fläche auf Grund nicht exakter Frequenzen tatsächlich schichtförmig. Da die Magnetfeldlinien 34 nicht exakt parallel zur zweiten Gehäuseseite 40 verlaufen, ist somit auch der sensitive Bereich 38 folglich entsprechend der Magnetfeldlinien gekrümmt. Die Krümmung und Ausformung des ersten Magnetfelds 34 und damit des sensitiven Bereichs 38 kann unter Verwendung weiterer Mittel, beispielsweise einer Shim-Spule 56 und einer magnetischen Schirmung 58, beeinflusst und insbesondere homogenisiert werden.

Die Oberfläche 40 des Gehäuses 12 des Messgeräts 10' stellt in diesem Ausführungsbeispiel eine ebene Fläche dar, an der das zu untersuchende Öl 42b und/oder der Kraftstoff 42a und/oder die zu untersuchende Hydraulikflüssigkeit 42c angelegt werden kann.

In Figur 4b sind in schematischer Schnittdarstellung eines Details einer alternativen Ausführungsform eines Messgeräts 10, insbesondere des handgehaltenen Messgeräts 10', der Kernspinresonanz-Sensor 32 zusammen mit einem zu untersuchenden Öl 42b und/oder Kraftstoff 42a und/oder einer zu untersuchenden Hydraulikflüssigkeit 42c dargestellt. Dabei ist das durch die erste Vorrichtung, hier zwei parallel zur zweiten Gehäuseseite und kollinear angeordnete Permanentmagnete 46,46' (in Nord-Süd/Nord-Süd-Abfolge), erzeugte erste, insbesondere statische, Magnetfeld 34 im Wesentlichen parallel zu der zweiten Gehäuseseite 40 des Messgeräts 10'. Das durch die zweite Vorrichtung, hier eine Hochfrequenzspule 48, erzeugte zweite Magnetfeld 36 ist im Wesentlichen senkrecht zu dem ersten Magnetfeld 34 ausgerichtet. In einer alternativen Ausführungsform kann das Magnetfeld 34 auch mittels eines Elektromagneten erzeugt werden. Zwischen den beiden Permanentmagneten 46,46' befindet sich die Hochfrequenzspule 48, deren Wicklungsebene kollinear zur Erstreckungsrichtung der Permanentmagnete 46,46' und parallel zur zweiten Gehäuseseite 40 liegt. Diese Anordnung ist in unmittelbarer Nähe zur zweiten Gehäuseseite 40 im Innern des Messgeräts 10' positioniert. Sobald durch diese Spule Strom fließt, wird ein elektromagnetisches Feld, insbesondere das zweite Magnetfeld 36, induziert. Die beiden Magnetfelder überlagern sich in einem Bereich, der im Wesentlichen außerhalb des Gehäuses 12 des Messgeräts 10' liegt. Der sensitive Bereich 38 des Kernspinresonanz-Sensors 32 liegt ebenfalls in dem Überlagerungsfeld der Magnetfelder 34 und 36. In Abhängigkeit der Frequenz des eingestrahlten elektromagnetischen Felds 36 und der statischen Magnetfeldstärke des ersten Magnetfelds 34 wird der sensitive Bereich im Idealfall durch eine Fläche definiert, auf der die Magnetfeldstärke des ersten Magnetfelds 34 konstant ist und insbesondere einen definierten Betrag aufweist. In Realität ist die Fläche auf Grund nicht exakter Frequenzen tatsächlich schichtförmig. Da die Magnetfeldlinien 34 nicht exakt parallel zur zweiten Gehäuseseite 40 verlaufen, ist somit auch der sensitive Bereich 38 folglich entsprechend der Magnetfeldlinien gekrümmt. Die Krümmung und Ausformung des ersten Magnetfelds 34 und damit des sensitiven Bereichs 38 kann unter Verwendung weiterer Mittel, beispielsweise einer Shim-Spule 56 und einer magnetischen Schirmung 58, beeinflusst und insbesondere homogenisiert werden.

Die Oberfläche 40 des Gehäuses 12 des Messgeräts 10' stellt in diesem Ausführungsbeispiel keine ebene Fläche dar, sondern weist eine speziell zur Aufnahme eines zu untersuchenden Kraftstoffs 42a und/oder Öls 42b und/oder einer zu untersuchenden Hydraulikflüssigkeit 42c ausgeformte Aufnahme 50 auf. Die Aufnahme 50 sowie die Anordnung des Kernspinresonanz-Sensors 32 sind derart aufeinander abgestimmt, dass das Öl 42b und/oder der Kraftstoff 42a und/oder die Hydraulikflüssigkeit 42c, beispielsweise in einem Behältnis befindlich, in die Aufnahme 50 eingeführt wird. Der sensitive Bereich 38 des Kernspinresonanz-Sensors 32 kommt dabei unmittelbar im Öl 42b und/oder im Kraftstoff 42a und/oder in der Hydraulikflüssigkeit 42c zu liegen.

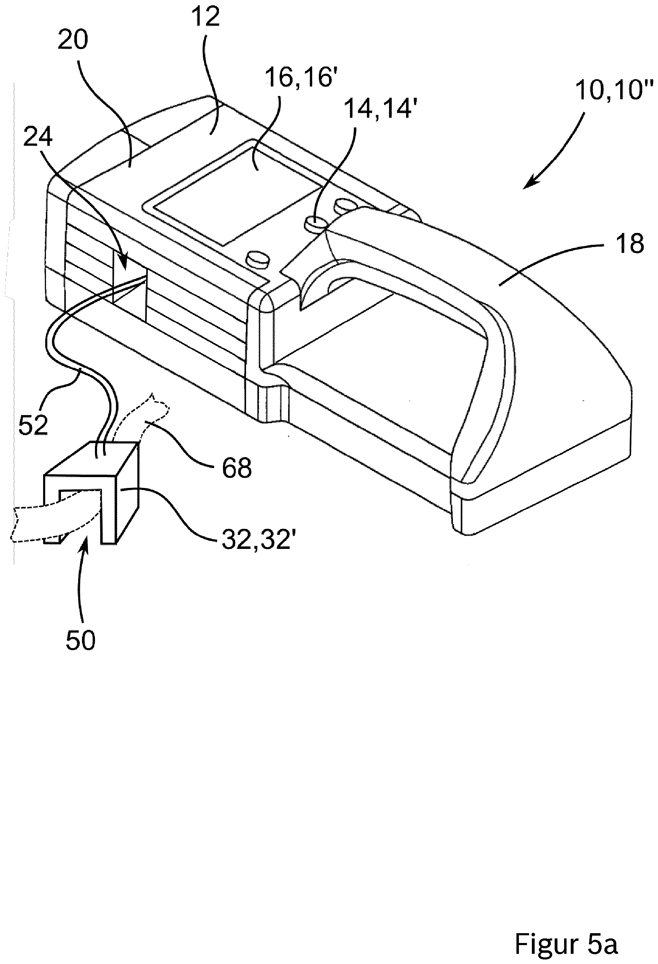

In Figur 5a ist ein alternatives oder zusätzliches Ausführungsbeispiel eines Messgeräts 10 in perspektivischer Darstellung gezeigt. Das dargestellte Messgerät 10" ist in diesem Ausführungsbeispiel sowohl als ein handgehaltenes, energieautonomes Messgerät verwendbar, als auch in einen Motorraum eines Kraftfahrzeugs integrierbar. Insbesondere gelten die für die Ausführungsbeispiele der Figuren 1 bis 4b gemachten Aussagen im übertragenen Sinne auch für dieses Ausführungsbeispiel.

Das Messgerät 10" weist gegenüber dem in Figur 1 dargestellten Messgerät 10' einen Kernspinresonanz-Sensor 32 auf, der in einem kleinen Messkopf 32' untergebracht ist, der über ein Kabel 52 mit dem Messgerät 10' verbunden ist. Der Messkopf 32' kann zum Transport des Messgeräts 10' in einer Aussparung 24 des Gehäuses 12 des Messgeräts 10' verstaut werden. Der Messkopf 32' weist eine C-förmige Aufnahme 50 auf, mittels der der Messkopf 32' auf ein kraftstoff- und/oder ölführendes und/oder hydraulikflüssigkeitführendes Bauteil eines Kraftfahrzeugs platziert und dort befestigt werden kann. In der Figur 5a ist der Messkopf 32' beispielhaft an einem Schlauch 68, beispielsweise einem Bremsschlauch, befestigt.

In Figur 5b ist in einer schematischen Schnittdarstellung eines Ausführungsbeispiels des Messkopfes 32' der Kernspinresonanz-Sensor 32 zusammen mit einem zu untersuchenden Öl 42b und/oder Kraftstoff 42a und/oder einer zu untersuchenden Hydraulikflüssigkeit 42c - hier beispielsweise einer zu untersuchenden Bremsflüssigkeit - dargestellt, die sich in dem Schlauch 68 befindet. Der Messkopf 32' weist eine im Wesentlichen C-förmige Gestalt mit einer Aufnahme 50 auf, die auf zwei gegenüberliegenden Seiten offen ist. Auf diese Weise kann die Aufnahme 50 des Messkopfes 32' derart auf den Schlauch 68 positioniert und über Fixierelemente (hier nicht näher dargestellt) fixiert werden, dass der Schlauch 68 entlang der Richtung, die durch die beiden offenen Seiten der Aufnahme 50 gegeben ist, verläuft. Zwei kollinear angeordnete Permanentmagnete 46,46', in Nord-Süd/Nord-Süd-Abfolge ausgerichtet, erzeugen ein erstes, insbesondere statisches, Magnetfeld 34, das die Aufnahme 50 im Wesentlichen homogen durchsetzt. Dieses zur Ausrichtung der Kernspins der in dem untersuchten Öl 42b und/oder Kraftstoff 42a und/oder in der untersuchten Hydraulikflüssigkeit 42c vorhandenen Atomkerne vorgesehene erste Magnetfeld 34 weist beispielhaft insbesondere eine Magnetfeldstärke von 0.5 Tesla auf, wobei die Permanentmagnete 46,46' in diesem Ausführungsbeispiel aus einer Neodym-Eisen-Bor-Legierung hergestellt sind. In einer alternativen Ausführungsform kann das Magnetfeld 34 auch mittels eines Elektromagneten erzeugt werden. Die zweite Vorrichtung zur Erzeugung des zweiten Magnetfeldes wird in diesem Ausführungsbeispiel durch eine Hochfrequenzspule 48 in Form einer platzsparenden Sattelspule gebildet. Sobald durch diese Spule Strom fließt, wird ein elektromagnetisches Feld, insbesondere das zweite Magnetfeld 36, induziert. Das durch die Hochfrequenzspule 48 erzeugte zweite Magnetfeld 36 ist im Wesentlichen senkrecht zu dem ersten Magnetfeld 34 ausgerichtet, wobei die beiden Magnetfelder 34, 36 sich in einem Bereich, der im Wesentlichen außerhalb des Gehäuses 12, insbesondere außerhalb der Gehäuseoberfläche 60 des Messkopfes 32', des Messkopfes 32', jedoch innerhalb der Aufnahme 50 des Messkopfes 32', liegt. Der sensitive Bereich 38 des Kernspinresonanz-Sensors 32 liegt ebenfalls in dem Überlagerungsfeld der Magnetfelder 34 und 36 und somit innerhalb der Aufnahme 50, außerhalb der Oberfläche 66 des Messkopfs 32'. In Abhängigkeit der Frequenz des eingestrahlten elektromagnetischen Felds 36 und der statischen Magnetfeldstärke des ersten Magnetfelds 34 wird der sensitive Bereich im Idealfall durch eine Fläche definiert, auf der die Magnetfeldstärke des ersten Magnetfelds 34 konstant ist und insbesondere einen definierten Betrag aufweist. In Realität ist die Fläche auf Grund nicht exakter Frequenzen tatsächlich schichtförmig. Da die Magnetfeldlinien 34 nicht exakt parallel verlaufen, ist somit auch der sensitive Bereich 38 folglich entsprechend der Magnetfeldlinien gekrümmt. Die Krümmung und Ausformung des ersten Magnetfelds 34 und damit des sensitiven Bereichs 38 kann unter Verwendung weiterer Mittel, beispielsweise einer Shim-Spule 56 und einer magnetischen Schirmung 58, beeinflusst und insbesondere homogenisiert werden.

Ferner ist denkbar, den Kernspinresonanz-Sensor 32 direkt an oder in einem Motor, an Anbauaggregaten eines Motors, in einem Ölmessstab, in einem öl-und/oder kraftstoff- und/oder hydraulikflüssigkeitführenden Rohr oder Schlauch zu integrieren. Beispielsweise kann der Messkopf 32' des Kernspinresonanz-Sensor 32 mit einem Gewinde 70 ausgeführt sein, wobei mittels des Gewindes 70 eine Fixierung im öl- und/oder kraftstoff- und/oder hydraulikflüsssigkeitführenden Bauteil 72 eines Fahrzeugs realisierbar ist. Derart kommen die vom Kernspinresonanz-Sensor 32 erzeugten Magnetfelder 34,36 und insbesondere der sensitive Bereich 38 im Öl 42b und/oder Kraftstoff 42a und/oder in der Hydraulikflüssigkeit 42c des öl- und/oder kraftstoff- und/oder hydraulikflüsssigkeitführenden Bauteils zu liegen. In Figur 5c ist eine derartige Ausführungsform des Messkopfes 32' in einer weiteren schematischen Schnittdarstellung wiedergegeben.

Die mit dem Messgerät 10' ermittelten Auswerteergebnisse werden zur weiteren Verwendung zumindest an ein externes Gerät wie beispielsweise ein Motorsteuergerät oder eine Bremskraftverstärkersteuerung ausgegeben. Das Motorsteuergerät oder die Bremskraftverstärkersteuerung verwendet die ermittelten Informationen zur auf die real vorliegenden Eigenschaften des Öls 42b und/oder des Kraftstoffs 42a und/oder der Hydraulikflüssigkeit 42c (hier: Bremsflüssigkeitseigenschaften) angepassten und somit zur verbesserten, bevorzugt optimierten, Steuerung und/oder Regelung des Motors bzw. der Bremskraft während eines Bremsvorganges des Fahrzeugs.

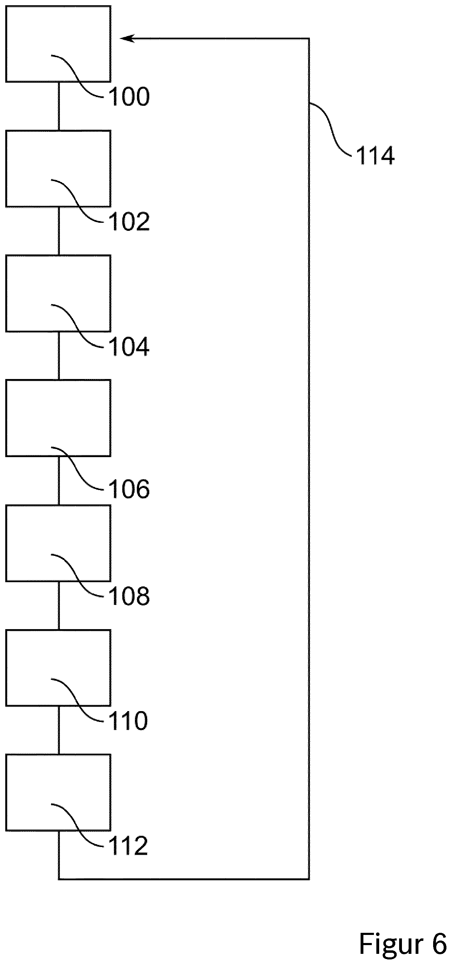

In der Figur 6 ist ein Verfahrensdiagramm gezeigt, dass ein Ausführungsbeispiel des Verfahrens zur Untersuchung von Öl 42b und/oder Kraftstoff 42a und/oder Hydraulikflüssigkeit 42c mittels eines Messgeräts 10, insbesondere mittels eines handgehaltenen Messgeräts 10', darstellt. In einem ersten Verfahrensschritt 100 wird das Messgerät 10 eingeschaltet und befindet sich nach einer kurzen Hochlaufzeit in einem Leerlaufmodus. Anschließend werden in einem optionalen Verfahrensschritt 102 unter Verwendung der Eingabevorrichtung 14 Angaben betreffend einen zu untersuchenden Kraftstoff 42a und/oder ein zu untersuchendes Öl 42b und/oder eine zu untersuchende Hydraulikflüssigkeit 42c spezifiziert. In Verfahrensschritt 104 findet eine Kalibrierung des Kernspinresonanz-Sensors 32 unter Verwendung einer geräteintern vorgesehenen Tetramethylsilan-Probe statt, in dessen Anschluss das Messgerät 10 für die Untersuchung des Kraftstoffs 42a und/oder Öls 42b und/oder der Hydraulikflüssigkeit 42c einsatzbereit ist. Zur Messung eines Kernspinresonanzsignals in dem Öl 42b und/oder in dem Kraftstoff 42a und/oder in der Hydraulikflüssigkeit 42c wird das Messgerät 10 in Verfahrensschritt 106 (optional) mit seiner zweiten Gehäuseseite 40 flächig in unmittelbarer Nähe zu dem Öl 42b und/oder Kraftstoff 42a und/oder der Hydraulikflüssigkeit 42c, insbesondere in Berührung zu dessen/deren Oberfläche 44, positioniert - hier beispielhaft entsprechend der geometrischen Ausführung des Messgeräts 10 nach dem Ausführungsbeispiel aus Figur 4a. Dabei dringen die durch den Kernspinresonanz-Sensor 32 erzeugten Magnetfelder 34,36 durch die zweite Gehäuseseite 40 aus dem Messgerät 10 aus und in das Öl 42b und/oder in den Kraftstoff 42a und/oder in die Hydraulikflüssigkeit 42c ein, wobei der sensitive Bereich 38 in dem Öl 42b und/oder in dem Kraftstoff 42a und/oder in der Hydraulikflüssigkeit 42c zu liegen kommt (siehe insbesondere die Figuren 3, 4a, 4b, 5b, 5c). Magnetfeldänderungen in Folge eines Kernspinresonanzeffekts der in dem Öl 42b und/oder Kraftstoff 42a und/oder in der Hydraulikflüssigkeit 42c angeregten Kernspins der Atomkerne, d.h. verursacht durch Absorption und/oder Emission elektromagnetischer Felder durch die Atomkerne einhergehend mit einer Änderung deren Energiezustände, wird mittels der Hochfrequenzspule 48 des Kernspinresonanz-Sensors 32 detektiert (Verfahrensschritt 108). Dieses Messsignal, insbesondere ein Messsignal repräsentierend ein Spektrum und/oder Relaxationskurven, wird an die Auswertevorrichtung 30 weitergeleitet, von der es mittels Auswerteroutinen aufbereitet, insbesondere gefiltert und/oder geglättet wird. Anschließend erfolgt eine Auswertung des Messsignals des Kernspinresonanz-Sensors durch Vergleich des Messsignals mit Referenzdaten einer Referenzdatenbank (Verfahrensschritt 110). Dabei werden die gemessenen Spektren und/oder Relaxationskurven und/oder Relaxationszeiten mit Referenzspektren bzw. Referenzrelaxationskurven bzw. Referenzrelaxationszeiten abgeglichen. Die Erkennung von Übereinstimmungen und/oder Abweichungen des Messsignals mit bzw. von den Referenzdaten erlaubt in diesem Verfahrensschritt die schnelle und präzise Auswertung des Messsignals, bei der Informationen betreffend das untersuchte Öl 42b und/oder den untersuchten Kraftstoff 42a und/oder die untersuchte Hydraulikflüssigkeit 42c erhalten und aufbereitet werden. Die Auswerteergebnisse, insbesondere die ermittelte Information und/oder eine Abweichung der ermittelten Information von Referenzdaten der Referenzdatenbank, werden anschließend mittels an die Ausgabevorrichtung 16 weitergeleitet (Verfahrensschritt 112). Das ausgewertete Messergebnis, d.h. die Information betreffend das untersuchte Öl 42b und/oder den untersuchten Kraftstoff 42a und/oder die untersuchte Hydraulikflüssigkeit 42c, kann dem Bediener des Messgeräts 10 auf dem Display 16' dargestellt und/oder über die Datenkommunikationsschnittstelle 54 an ein weiteres Datenverarbeitungsgerät gesendet werden. Die Ausgabe auf dem Display 16' kann dabei grafisch, numerisch und/oder alphanumerisch, beispielsweise in Form eines Messwerts, einer Messkurve, eines Signalverlaufs, eines Zeitverlauf, als Bilddaten oder in einer Gradientendarstellung sowie in einer Kombination derer erfolgen. Alternativ oder zusätzlich ist eine Darstellung mittels einer Signalanzeige möglich, insbesondere beispielsweise einer Leuchtdiode, die beispielsweise über eine Farbcodierung (z.B. rot, gelb, grün) eine Zielgröße bewertet.

Das Verfahren wiederholt sich bei der weiteren Untersuchung des Kraftstoffs 42a und/oder des Öls 42b und/oder der Hydraulikflüssigkeit 42c, angedeutet durch den Pfeil 114.

## Patentansprüche

1. Verwendung eines Messgeräts (10, 10', 10"), insbesondere eines handgehaltenen Messgeräts (10'), bevorzugt eines Messgeräts (10, 10', 10") mit einem Gehäuse (12), mit zumindest einem Kernspinresonanz-Sensor (32), einer Steuervorrichtung (28) zur Steuerung des Messgeräts (10, 10', 10"), einer Auswertevorrichtung (30) zur Auswertung eines von dem Kernspinresonanz-Sensor (32) gelieferten Messsignals, einer Ausgabevorrichtung (16, 16') zur Ausgabe einer ermittelten Information sowie mit einer Energieversorgungsvorrichtung (22), zur Untersuchung von Kraftstoff (42a) und/oder Öl (42b) und/oder Hydraulikflüssigkeit (42c) eines Fahrzeugs, wobei mittels der Auswertevorrichtung (30) des Messgeräts (10, 10', 10") das von dem Kernspinresonanz-Sensor (32) gelieferte Messsignal, insbesondere ein Spektrum und/oder eine Relaxationszeit des Messsignals resultierend aus der Anregung von Kernspins in dem zu untersuchenden Kraftstoff (42a) und/oder Öl (42b) und/oder der Hydraulikflüssigkeit (42c) durch den Kernspinresonanz-Sensor (32), ausgewertet und daraus eine Information abgeleitet wird, wobei diese mittels der Auswertevorrichtung (30) ermittelte Information zur Steuerung und/oder Regelung zumindest einer Komponente des Fahrzeugs verwendet wird, um die Steuerung bzw. Regelung der zumindest einen Komponente zu verbessern.

2. Verwendung eines Messgeräts (10, 10', 10") nach Anspruch 1, wobei mittels der Auswertevorrichtung (30) des Messgeräts (10, 10', 10") die ermittelte Information, insbesondere ein Spektrum und/oder eine Relaxationszeit, mit Referenzdaten einer Referenzdatenbank verglichen wird.

3. Verwendung eines Messgeräts (10, 10', 10") nach einem der Ansprüche 1-2, wobei mittels einer Ausgabevorrichtung (16, 16') des Messgeräts (10, 10', 10"), insbesondere mittels eines Displays (16'), eine ermittelte Information, insbesondere ein Spektrum und/oder eine Relaxationszeit, und/oder eine Abweichung einer ermittelten Information von Referenzdaten einer Referenzdatenbank ausgegeben werden/wird, insbesondere dargestellt werden/wird.

4. Verwendung eines Messgeräts (10, 10', 10") nach einem der Ansprüche 1-3, wobei vor Untersuchung des Kraftstoffes (42a) und/oder Öls (42b) und/oder der Hydraulikflüssigkeit (42c) unter Verwendung einer geräteintern vorgesehenen Standardprobe, insbesondere unter Verwendung einer geräteintern vorgesehenen Tetramethylsilan-Probe, eine Kalibrierung des Messgeräts (10, 10', 10") durchführt wird.

5. Verwendung eines Messgeräts (10, 10', 10") nach einem der Ansprüche 1-4 in einem Kraftfahrzeug, bevorzugt während eines Betriebs des Kraftfahrzeugs.

6. Verwendung eines Messgeräts (10, 10', 10") nach Anspruch 5 unmittelbar an einem kraftstoffführenden und/oder ölführenden und/oder hydraulikflüssigkeitsführenden Bauteil (72) des Fahrzeugs.

7. Verwendung eines Messgeräts (10, 10', 10") nach einem der Ansprüche 5-6, wobei eine ermittelte Information, insbesondere ein Spektrum und/oder eine Relaxationszeit, und/oder eine Abweichung einer ermittelten Information von Referenzdaten einer Referenzdatenbank, an und/oder in einem Fahrerstand ausgegeben werden/wird, insbesondere dargestellt werden/wird.

8. Fahrzeug umfassend ein Messgerät (10, 10', 10") mit zumindest
• einem Kernspinresonanz-Sensor (32),
• einer Steuervorrichtung (28) zur Steuerung des Messgeräts (10, 10', 10"),
• einer Auswertevorrichtung (30) zur Auswertung eines von dem Kernspinresonanz-Sensor (32) gelieferten Messsignals,
• einer Ausgabevorrichtung (16, 16') zur Ausgabe von ermittelten Informationen sowie
sowie
• einer Energieversorgungsvorrichtung (22), wobei das Messgerät (10, 10', 10") zur Untersuchung von Kraftstoff (42a) und/oder Öl (42b) und/oder Hydraulikflüssigkeit (42c) vorgesehen ist, wobei die Auswertevorrichtung (30) des Messgeräts (10, 10', 10") dazu eingerichtet ist, das von dem Kernspinresonanz-Sensor (32) gelieferte Messsignal, insbesondere ein Spektrum und/oder eine Relaxationszeit des Messsignals resultierend aus der Anregung von Kernspins in dem zu untersuchenden Kraftstoff (42a) und/oder Öl (42b) und/oder Hydraulikflüssigkeit (42c) durch den Kernspinresonanz-Sensor (32), auszuwerten und daraus eine Information abzuleiten, und wobei das Fahrzeug dazu eingerichtet ist, die derart erhaltene Information an eine Komponente des Fahrzeugs zum Zwecke einer verbesserten Steuerung und/oder Regelung der Komponente des Fahrzeugs weiterzuleiten.

## Claims

1. Use of a measurement device (10, 10', 10"), in particular a hand-held measurement device (10'), preferably a measurement device (10, 10', 10") having a housing (12), comprising at least a nuclear magnetic resonance sensor (32), a control apparatus (28) for controlling the measurement device (10, 10', 10"), an evaluation apparatus (30) for evaluating a measurement signal supplied by the nuclear magnetic resonance sensor (32), an output apparatus (16, 16') for outputting ascertained information and an energy supply apparatus (22), for examining fuel (42a) and/or oil (42b) and/or hydraulic fluid (42c) of a vehicle, wherein the measurement signal supplied by the nuclear magnetic resonance sensor (32), in particular a spectrum and/or a relaxation time of the measurement signal resulting from the excitation of nuclear spins in the fuel (42a) and/or oil (42b) and/or hydraulic fluid (42c) to be examined by way of the nuclear magnetic resonance sensor (32), is evaluated by means of the evaluation apparatus (30) of the measurement device (10, 10', 10"), and information is derived therefrom, wherein this information ascertained by means of the evaluation apparatus (30) is used for controlling with open-loop and/or closed-loop control at least one component of the vehicle in order to improve open-loop and/or closed-loop control of the at least one component.

2. Use of a measurement device (10, 10', 10") according to Claim 1, wherein the ascertained information, in particular a spectrum and/or a relaxation time, is compared with reference data from a reference database by means of the evaluation apparatus (30) of the measurement device (10, 10', 10").

3. Use of a measurement device (10, 10', 10") according to one of Claims 1-2, wherein ascertained information, in particular a spectrum and/or a relaxation time, and/or a deviation of ascertained information from reference data from a reference database is output, in particular displayed, by means of an output apparatus (16, 16') of the measurement device (10, 10', 10"), in particular by means of a display (16').

4. Use of a measurement device (10, 10', 10") according to one of Claims 1-3, wherein the measurement device (10, 10', 10") is calibrated using a standard sample provided within the device, in particular using a tetramethylsilane sample provided within the device, before examining the fuel (42a) and/or oil (42b) and/or hydraulic fluid (42c).

5. Use of a measurement device (10, 10', 10") according to one of Claims 1-4 in a motor vehicle, preferably during operation of the motor vehicle.

6. Use of a measurement device (10, 10', 10") according to Claim 5 directly at a fuel-conducting and/or oil-conducting and/or hydraulic-fluid-conducting component (72) of the vehicle.

7. Use of a measurement device (10, 10', 10") according to one of Claims 5-6, wherein ascertained information, in particular a spectrum and/or a relaxation time, and/or a deviation of ascertained information from reference data in a reference database, is output, in particular displayed, to and/or in an operator's platform.

8. Vehicle comprising a measurement device (10, 10', 10"), comprising at least
• a nuclear magnetic resonance sensor (32),
• a control apparatus (28) for controlling the measurement device (10, 10', 10"),
• an evaluation apparatus (30) for evaluating a measurement signal supplied by the nuclear magnetic resonance sensor (32),
• an output apparatus (16, 16') for outputting ascertained information, and
• an energy supply apparatus (22),
wherein the measurement device (10, 10', 10") is provided for examining fuel (42a) and/or oil (42b) and/or hydraulic fluid (42c), wherein the evaluation apparatus (30) of the measurement device (10, 10', 10") is configured to evaluate the measurement signal supplied by the nuclear magnetic resonance sensor (32), in particular a spectrum and/or a relaxation time of the measurement signal resulting from the excitation of nuclear spins in the fuel (42a) and/or oil (42b) and/or hydraulic fluid (42c) to be examined by way of the nuclear magnetic resonance sensor (32), and to derive therefrom information, and wherein the vehicle is configured to pass on the information thus obtained to a component of the vehicle for the purpose of improved open-loop and/or closed-loop control of the component of the vehicle.

## Revendications

1. Utilisation d'un appareil de mesure (10, 10', 10"), en particulier d'un appareil de mesure portatif (10'), de préférence d'un appareil de mesure (10, 10', 10") doté d'un boîtier (12), comprenant au moins un capteur à résonance magnétique nucléaire (32), un dispositif de commande (28) pour commander l'appareil de mesure (10, 10', 10"), un dispositif d'évaluation (30) pour évaluer un signal de mesure fourni par le capteur à résonance magnétique nucléaire (32), un dispositif de sortie (16, 16') pour sortir une information déterminée, ainsi qu'un dispositif d'alimentation en énergie (22) pour analyser le carburant (42a) et/ou l'huile (42b) et/ou le liquide hydraulique (42c) d'un véhicule,
dans laquelle, au moyen du dispositif d'évaluation (30) de l'appareil de mesure (10, 10', 10"), le signal de mesure fourni, en particulier un spectre et/ou un temps de relaxation du signal de mesure résultant de l'excitation du spin nucléaire dans le carburant (42a) et/ou l'huile (42b) et/ou le liquide hydraulique (42c) à analyser par le capteur à résonance magnétique nucléaire (32), est évalué par le capteur à résonance magnétique nucléaire (32) et une information en est déduite, cette information déterminée au moyen du dispositif d'évaluation (30) étant utilisée pour commander et/ou réguler au moins un composant du véhicule afin d'améliorer la commande ou la régulation du au moins un composant.

2. Utilisation d'un appareil de mesure (10, 10', 10") selon la revendication 1, dans laquelle, au moyen du dispositif d'évaluation (30) de l'appareil de mesure (10, 10', 10"), l'information déterminée, en particulier un spectre et/ou un temps de relaxation, est comparée à des données de référence d'une base de données de référence.

3. Utilisation d'un appareil de mesure (10, 10', 10") selon l'une quelconque des revendications 1 à 2, dans laquelle, au moyen d'un dispositif de sortie (16, 16') de l'appareil de mesure (10, 10', 10"), en particulier au moyen d'un affichage (16'), une information déterminée, en particulier un spectre et/ou un temps de relaxation, et/ou un écart d'une information déterminée par rapport à des données de référence d'une base de données de référence sont sortis, en particulier représentés.

4. Utilisation d'un appareil de mesure (10, 10', 10") selon l'une quelconque des revendications 1 à 3, dans laquelle avant l'analyse du carburant (42a) et/ou de l'huile (42b) et/ou du liquide hydraulique (42c), en utilisant un échantillon standard prévu de façon interne à l'appareil, en particulier en utilisant un échantillon de tétraméthylsilane prévu de façon interne à l'appareil, un étalonnage de l'appareil de mesure (10, 10', 10") est effectué.

5. Utilisation d'un appareil de mesure (10, 10', 10") selon l'une quelconque des revendications 1 à 4 dans un véhicule automobile, de préférence pendant un fonctionnement du véhicule automobile.

6. Utilisation d'un appareil de mesure (10, 10', 10") selon la revendication 5 directement au niveau d'un composant (72) transportant du carburant et/ou transportant de l'huile et/ou transportant un liquide hydraulique du véhicule.

7. Utilisation d'un appareil de mesure (10, 10', 10") selon l'une quelconque des revendications 5 à 6, dans laquelle une information déterminée, en particulier un spectre et/ou un temps de relaxation, et/ou un écart d'une information déterminée par rapport à des données de référence d'une base de données de référence, sont sortis, en particulier représentés, à et/ou dans un poste de conduite.

8. Véhicule, comprenant un appareil de mesure (10, 10', 10") comprenant au moins
• un capteur à résonance magnétique nucléaire (32),
• un dispositif de commande (28) pour commander l'appareil de mesure (10, 10', 10"),
• un dispositif d'évaluation (30) pour évaluer un signal de mesure fourni par le capteur à résonance magnétique nucléaire (32),
• un dispositif de sortie (16, 16') pour sortir des informations déterminées, et
• un dispositif d'alimentation en énergie (22),
l'appareil de mesure (10, 10', 10") étant prévu pour analyser le carburant (42a) et/ou l'huile (42b) et/ou le liquide hydraulique (42c), le dispositif d'évaluation (30) de l'appareil de mesure (10, 10', 10") étant aménagé pour évaluer le signal de mesure fourni par le capteur à résonance magnétique nucléaire (32), en particulier un spectre et/ou un temps de relaxation du signal de mesure résultant de l'excitation du spin nucléaire dans le carburant (42a) et/ou l'huile (42b) et/ou le liquide hydraulique (42c) à analyser par le capteur à résonance magnétique nucléaire (32), et pour en déduire une information, et le véhicule étant aménagé pour retransmettre l'information ainsi obtenue à un composant du véhicule en vue d'une commande et/ou d'une régulation améliorée(s).
